# EUROPEAN PATENT SPECIFICATION

(11) **EP 0 818 443 B1**
(45) Date of publication and mention of the grant of the patent: **05.12.2001**
(21) Application number: 97305020.6
(22) Date of filing: 09.07.1997
(51) Int. Cl.: C07C 323/60, C07D 307/32, C07F 7/18, C07C 323/52, A61K 31/16

(54) **Mercaptoketones and mercaptoalcohols, a process for their preparation and their use as inhibitors of matrix metalloproteinases**
Mercaptoketone und Mercaptoalkohole, Verfahren zu ihrer Herstellung und ihre Anwendung als Matrix-Metalloproteinase-Inhibitoren
Mercaptocétones et mercaptoalcools, leur procédé de préparation et leur utilisation comme inhibiteurs de métalloprotéinases matricielles

(30) Priority: 10.07.1996 US 677994
(43) Date of publication of application: 14.01.1998
(73) Proprietor: American Cyanamid Company, Madison, New Jersey 07940-0874 (US)
(72) Inventor: Levin, Jeremy Ian, Nanuet, New York 10954 (US)
(74) Representative: Wileman, David Francis, Dr.

(56) References cited:
- WO-A-94/07481
- WO-A-94/25435
- WO-A-96/40738

## Description

This invention relates to mercaptoketone and diastereomeric mercaptoalcohol derivatives of Formula I useful as matrix metalloproteinase inhibitors and a process for the stereospecific synthesis of each of the diasteromers. Unexpectedly, the mercaptoalcohols with the *S*-configuration at the hydroxyl-bearing carbon have been found to be at least 4 times more potent than the analogous (R)-alcohols both *in vitro* and *in vivo.*

### Background of the Invention

Matrix metalloproteinases (MMP) are a family of zinc-containing calcium dependent proteinases, including stromelysins, collagenases, and gelatinases. These MMP enzymes are capable of degrading the proteinaceous components of connective tissue and appear to be involved in tissue remodeling, i.e., wound healing and connective tissue turnover. Approximately thirteen MMPs have been identified. The collagenases cleave fibrillar collagen. The stromelysins degrade fibronectin, laminin, and proteoglycans in addition to collagen. The gelatinases can degrade denatured collagen (gelatin) and type IV collagen, the major component of basement membranes.

Elevated levels of collagenase and stromelysin are associated with both osteo- and rheumatoid arthritis, having been observed both in synovium and cartilage in amounts proportional to the severity of the disease. The gelatinases are thought to play a key role in tumor metastasis, since they degrade the basement membrane through which tumor cells must pass in order to migrate away from the primary tumor site and thus enable the migration from the primary site. Gelatinase is also associated with the process of angiogenesis which is essential for the growth of solid tumors. In addition to the association with osteo- and rheumatoid arthritis and tumor metastasis, MMPs are implicated in corneal ulceration, gingivitis, multiple sclerosis and other neurological disorders, and emphysema (Beeley et al., *Curr. Opin. Ther. Patents* **4**(1), 7-16 (1994)).

Compounds which bind zinc at the active site of the enzyme prevent the catalytic activity of MMPs. MMP inhibitor activity has been found in certain peptidyl hydroxamic acids, peptidylalkyl carboxylic acids, peptidylphosphinic and phosphonic acids. Furthermore, peptidyl thiols incorporating a carbonyl group two atoms removed from the thiol group have been shown to be potent MMP inhibitors. For example, in the compound below, as disclosed in *Journal of Medicinal Chemistry* **36,** 4030-4039 (1993), the SSR 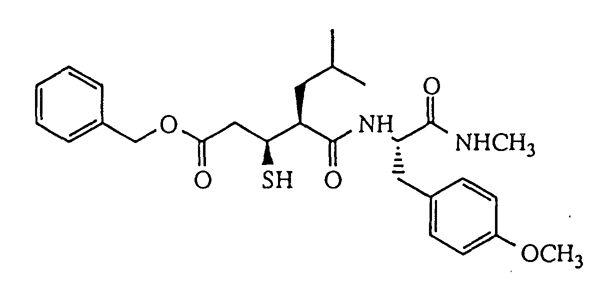 isomer is a potent (IC₅₀=2nM) inhibitor of human collagenase (J. R. Morphy et al., *Current Medicinal Chemistry* **2**,743-762 (1995).

Merck discloses the compound 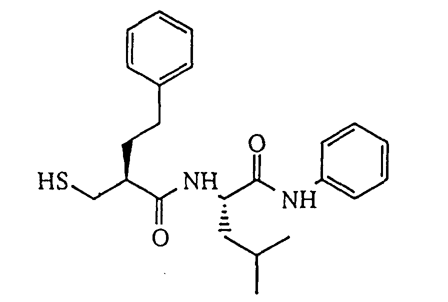 in the PCT application WO 9407481 as a moderate inhibitor of the MMP stromelysin. The compound 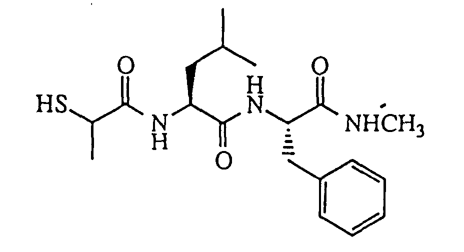 is disclosed in the PCT application WO 9513289. The mercapto sulfide MMP inhibitor compound below was disclosed in the PCT application WO 9509833. 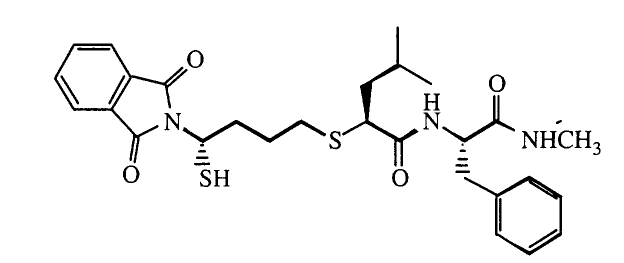 MMP inhibiting compounds of the formula 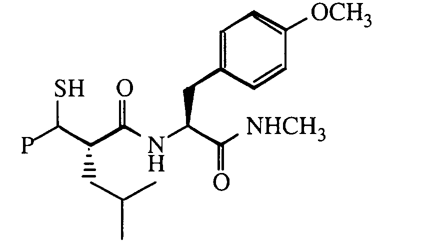 where P is H, methyl or phenyl are disclosed by Donald et al., US patent 4,595,700. The compound where P is 2-oxopropyl is approximately 20 times more potent than the compound where P is methyl which is in turn about 20 times more potent than the compound where P is hydrogen (EP 0322,184 A2). In all of the above noted examples the thiol moiety is two carbons removed from an amide carbonyl group. WO 94/25435 discloses a broad class of metalloproteases.

The matrix metalloproteinase inhibiting compounds of this invention are represented by formula I 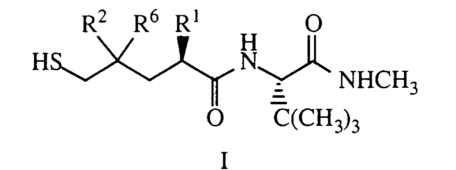 where R¹ is C₁-C₁₂ alkyl, straight or branched and optionally substituted by halogen, hydroxy, C₁-C₆ alkoxy, amino, carboxyl, C₁-C₆ alkoxycarbonyl, carboxamido, nitrile, mono- or di-(C₁-C₆)alkylamino, thio, C₁-C₆ alkylthio, aryl, -Oaryl or -OCH₂aryl where aryl is optionally substituted with C₁-C₆ alkyl, C₁-C₆ alkoxy, carboxy, halogen, cyano, nitro, carboxamido, or hydroxy; and C₁-C₆ alkanesulfonyloxy. R² is α-OH or β-OH and R⁶ is H or R² and R⁶ together forms carbonyl. In the above definition, aryl is a 5 to 10 membered carbocyclic or heterocyclic mono or bicyclic aromatic group. When aryl is a heterocyclic group the heteroatom may be selected from one or more heteroatoms selected from oxygen, sulphur and nitrogen, e.g. one to three such heteroatoms which may be the same or different. The aryl group may suitably be selected from benzene, furan, thiophene, imidazole, naphthalene, quinoline, indole, benzothiophene, benzimidazole, pyridine, pyrimidine and benzofuran. This invention also encompasses all of the chemical intermediates required for the synthesis of compounds of formula I.

The diastereomeric compounds of formula I have been shown to inhibit the matrix metalloproteinases collagenase, stromelysin and gelatinase, both *in vitro* and *in vivo.* and are thus expected to be useful in the treatment of arthritis, corneal ulceration, multiple sclerosis, gingivitis, emphysema, inhibition of solid tumor growth, and prevention of tumor metastasis. The stereospecific synthesis of the mercaptoalcohols is desirable since the alcohols of the (*S*)-configuration (formula I, R² is β-OH) have been found to be at least 4 times more potent than the corresponding (*R*)-configured diastereomers both *in vitro* and *in vivo.* Disclosed herein is a process by which either of the two diastereomers of compounds of formula I (R² is α-OH or β-OH) can be synthesized via intermediate lactone diastereomers. Also disclosed herein are diastereomerically and entaniomerically pure intermediates and processes for their preparation.

The process for the sterospecific synthesis of each of the diastereomeric alcohols and ketones of Formula I is shown in Schemes I, II, and III. Schemes II and III outline alternate synthesis of diastereomeric furanone intermediates. The Roman numerals following the names of the compounds refer to structures shown in Schemes I, II, and III. Those skilled in the art of organic synthesis will recognize that stereochemical directing groups other than (*S*)-(-)-4-benzyl-2-oxazolidinone (A) as shown in Scheme I, including, but not limited to, alternatively substituted oxazolidinones, ephedrine derivatives and chiral 2,10-camphorsultams may be used to obtain the same results. Also, hydroxyl and mercaptan protecting groups other than those used herein in the following specific examples can be utilized. Thiol protecting groups, W, which can be used include phenyl, -C(O)aryl where aryl is as defined and optionally substituted as above, -C(O)C₁-C₁₂ alkyl, -CR³R⁴R⁵ where R³, R⁴ and R⁵ are independently H, methyl, -OC₁-C₁₂alkyl, O-tetrahydropyranyl, -S-benzyl, and phenyl optionally substituted by methoxy, hydroxy, nitro, or methyl; disulfides or any other group suitable for protecting sulfur. Thiol protecting groups Z includes H, phenyl, -CR³R⁴R⁵ where R³, R⁴ and R⁵ are independently H, methyl, -OC₁-C₁₂alkyl, O-tetrahydropyranyl, -S-benzyl, and phenyl optionally substituted by methoxy, hydroxy, nitro, or methyl; disulfides or any other group suitable for protecting sulfur. Hydroxyl protecting groups Y, include trimethylsilyl, t-butyldimethylsilyl, triethylsilyl, i-propyldimethylsilyl, trityldimethylsilyl, t-butyldiphenylsilyl, methyldi-i-propylsilyl, methyldi-t-butylsilyl, tri-i-propylsilyl, triphenylsilyl, benzyl, benzyl optionally substituted with methoxy, nitro, halo, cyano; and substituted methyl and ethyl ethers including triphenylmethyl, methoxymethyl, methoxyethoxymethyl, tetrahydropyran and allyl. Still other suitable oxygen and sulfur protecting groups as described in "Protective Groups in Organic Synthesis" (2nd ed., T.W. Greene and P.G.M. Wuts, John Wiley & Sons, New York, NY, 1991) may be used. Additionally, it will be apparent to those skilled in the art of organic synthesis that removal of the various chiral auxiliaries and mercapto and hydroxyl protecting groups may require methods other than those used in Schemes I, II, and III. It is understood by those skilled in the art that the various functionalities present on the molecule must be consistent with the chemical transformations proposed. This will frequently necessitate judgment as to the order of synthetic steps, protecting groups and deprotecting conditions.

Invention compounds and intermediates where R¹ is optionally substituted can be prepared following the steps outlined in schemes I, II, and III and following the procedures given in the examples starting with an appropriately substituted carboxylic acid of the formula R¹CH₂COOH or acid halide or anhydride thereof. Such acids are either commercially available or can be prepared according to standard literature procedures. Obviously, depending on the functionality of the substituent, standard protecting group chemistry may be required.

The formula I compounds of this invention where R² is α-OH (R configuration) or β-OH (S configuration) are prepared individually according to the process disclosed herein as outlined in Scheme I, starting with a common chiral acid 2(R)-2-R¹-pent-4-enoic acid of formula **i** . These 2-substituted pent-4-enoic acids are prepared according to literature procedures or by the sequence of reactions shown in Scheme I and in the following examples 1-6.

A synthetic scheme for preparing the starting acids (**i**) is shown in Scheme I. (4*S*)-4-Benzyl-3-((2*R*)-2-R¹-pent-4-enoyl)oxazolidin-2-ones, **B**, are prepared by acylation of (*S*)-(-)-4-benzyl-2-oxazolidinone, **A**, with an alkanoic acid halide of the formula R¹CH₂C(O)-halogen or the acid anhydride thereof, where R¹ is C₁-C₁₂ alkyl as defined above. A variety of N-acyl oxazolidinones, **A**, are known in the literature, including alkyl-O-CH₂aryl and alkyl-aryl derivatives which have previously been used for the synthesis of MMP inhibitors (Tomczuk, B. E. *et. al. Bioorg. & Med. Chem. Lett.* 5, 343, **1995**; Chapman, K.T., et. al. *Bioorg. & Med. Chem. Lett*. 6, 803, **1996**). The acyl chain is then alkylated stereospecifically α to the carbonyl with an allylating agent such as allyl halide or triflate, preferably allyl bromide, and the resulting (4*S*)-4-benzyl-3-((2*R*)-2-R¹-pent-4-enoyl)oxazolidin-2-one is hydrolyzed with lithium hydroperoxide or other suitable aqueous base to give the (2*R*)-2-R¹-penten-4-oic acids (**i**).

Reaction of the acid (i)with iodine affords predominantly the dihydrofuranone of formula **iib,** whereas reaction of the dimethylamide of the acid **i** with iodine affords predominantly the dihydrofuranone of formula **iia**. Either of the iodomethyldihydrofuranones **iia** or **iib** is converted to a protected thiol **(iiia** or **iiib)** by reaction with the anion of HSW, when appropriate, where W is as defined above, preferably by the sodium, lithium or potassium salt of thiolacetic acid. S-acetyl thiomethyldihydrofuranone **iiia** or **iii**b (W = Ac) is then hydrolyzed and the thiol protected as a cleavable thiol ether -SZ to give **iva** or **ivb**, where Z is defined as H, phenyl, -CR³R⁴R⁵ where R³, R⁴ and R⁵ are independently H, methyl, -OC₁-C₁₂alkyl, O-tetrahydropyranyl, -S-benzyl, and phenyl optionally substituted by methoxy, hydroxy, nitro, or methyl; disulfides or any other group suitable for protecting sulfur. The preferred protecting group Z for **iva** and **ivb** is the triphenylmethyl group which is readily formed from the thiol and trityl chloride in trifluoroacteic acid and is resistant to chemical reactions that occur in subsequent process steps.

Hydrolysis of the dihydrofuranone **iva** or **ivb** produces the S-protected 4-hydroxy-5-mercaptopentanoic acid **va** or **vb** respectively. The hydroxy group of the pentanoic acid **va** or **vb** is protected by a group Y by formation of an ether where Y is defined as H, trimethylsilyl, t-butyldimethylsilyl, triethylsilyl, i-propyldimethylsilyl, trityldimethylsilyl, t-butyldiphenylsilyl, , methyldi-i-propylsilyl, methyldi-t-butylsilyl, tri-i-propylsilyl, triphenylsilyl, benzyl, benzyl optionally substituted with methoxy, nitro, halogen or cyano, triphenylmethyl, methoxymethyl, methoxyethoxymethyl, tetrahydropyran and allyl. The preferred protecting group Y is the t-butyldimethylsilyl group.

A O,S-protected acid **via** or **vib** is coupled with 2(S)-t-butyl-N-methylglycine using standard amide coupling techniques to obtain the respective protected formula I compound **viia** or **viib**. Removal of the hydroxyl protecting group (Y) gives the S-protected formula I compound **viiia** or **viiib** respectively. Removal of the sulfur protecting group (Z) from **viiia** or **viiib** then gives a formula I compound where R² is β-OH (S configuration) or α-OH (R configuration) respectively. Either of the formula I compounds where R² is OH and R⁶ is H and Z is a protecting group can be oxidized followed by removal of the protecting group to give a compound of formula I where R² and R⁶ together form a carbonyl group.

Thioacetic acid ester **iiia** or **iiib** (W = Ac) is also available via the commercially available (R)-(-)- or (*S*)-(+)-dihydro-5-(hydroxymethyl)-2(3*H*)-furanone as shown in Scheme II. Thus, protection of the hydroxy group of the (S)-(+) enantiomer as the t-butyldimethylsilyl ether or any suitable bulky protecting group such as a trityl group, followed by deprotonation of the resulting lactone and alkylation of this anion with R¹X, where R¹ is as previously defined and X is a suitable leaving group such as halide or triflate, gives lactone **x**. Lactone **x** is also available via alkylation of a suitably substituted pseudoephedrine amide, or via the alkylation of an achiral amide enolate with an enantiomerically pure epoxide followed by acid-induced hydrolysis/equilibration (Myers, A.G., *et. al. J.* Org. *Chem.* 61, 2428, **1996**). Subsequent deprotection of the alcohol provides 2(*R*)-2-R¹-5(*S*)-5-hydroxymethyldihydrofuran-2-one(xia). (Lewis, C.N. *et. al. JCS Chem. Commun.* 1786, **1987**). This alcohol may be converted into lactonethioacetate **iiia** via a Mitsunobu-type reaction (Volante, R.P. *Tetrahedron Letters,* 22, 3119, **1981**) or by conversion of the alcohol into a suitable leaving group such as halide, triflate, mesylate or tosylate followed by displacement with thiolacetic acid or other suitable protected thiol equivalent and base. Using the same methodology (*R*)-(-)-dihydro-5-(hydroxymethyl)-2(3*H*)-furanone can be converted into thioacetate-lactone **iiib** via hydroxy-lactone intermediate **xib** (Scheme II).

Also, both diastereomeric lactones **iiia** and **iiib** can be synthesized from the pentenoic acid **i** as shown in Scheme III. Asymmetric dihydroxylation of the acid using Sharpless methodology (Sharpless, K.B., *et. al. Chem. Rev.* 94, 2483, **1994**) provides diol **xii** which then undergoes acid catalyzed lactonization to give either of lactone-alcohols **xia** or **xib**. The lactones are then converted into the corresponding thioacetate-lactones, **iiia** or **iiib** via the same methodology described above for the conversion of **iia** to **iiia** in Scheme II.

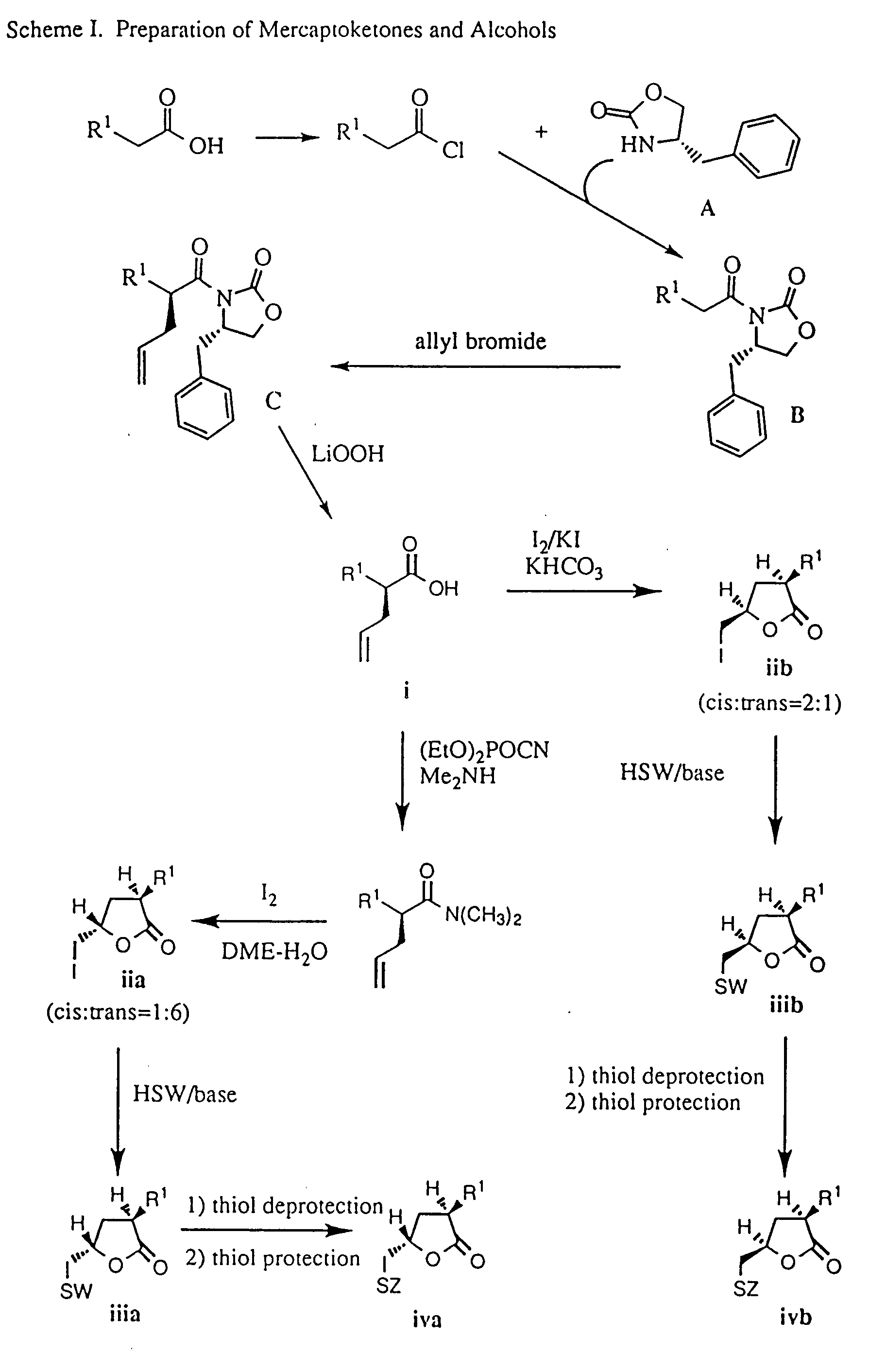

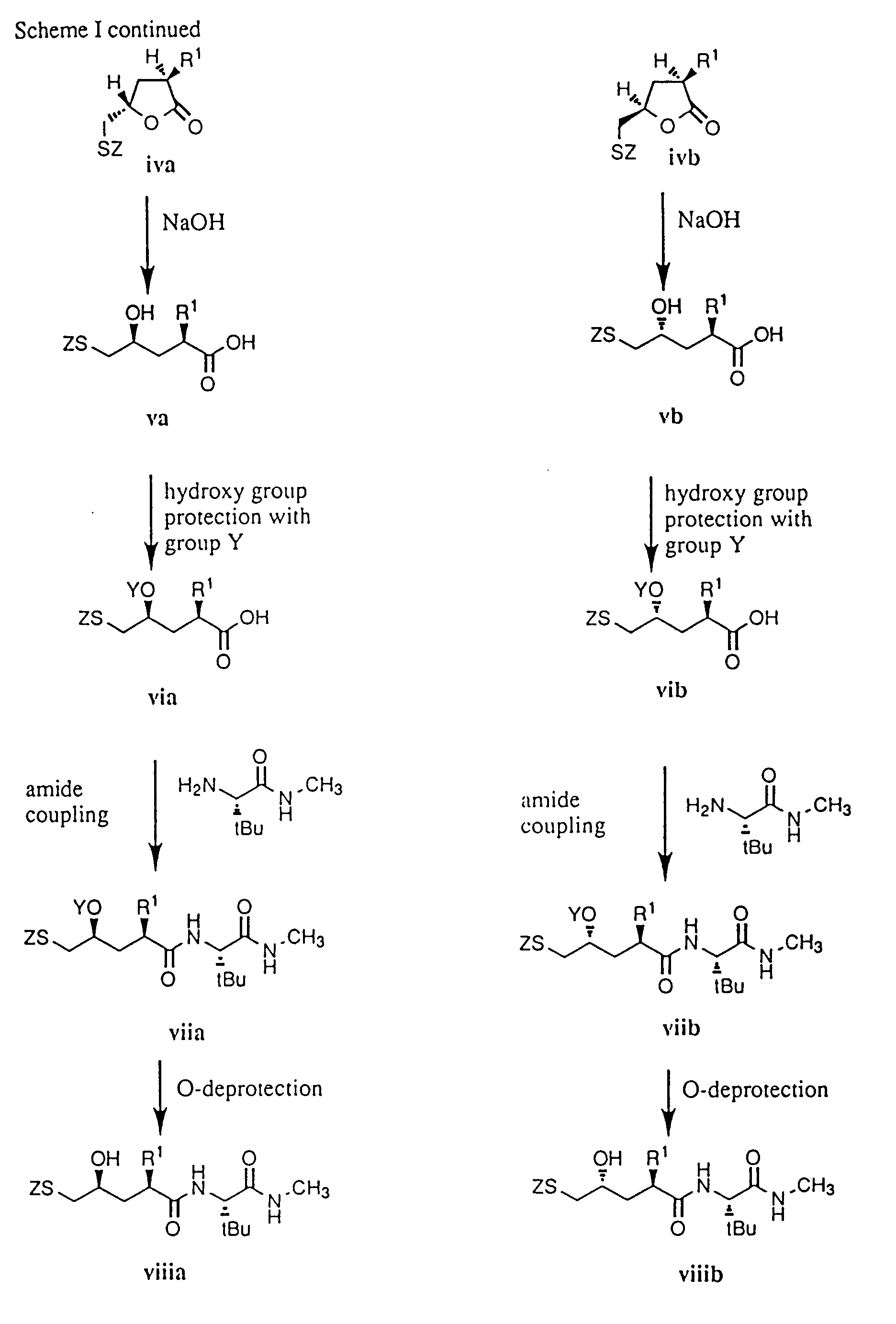

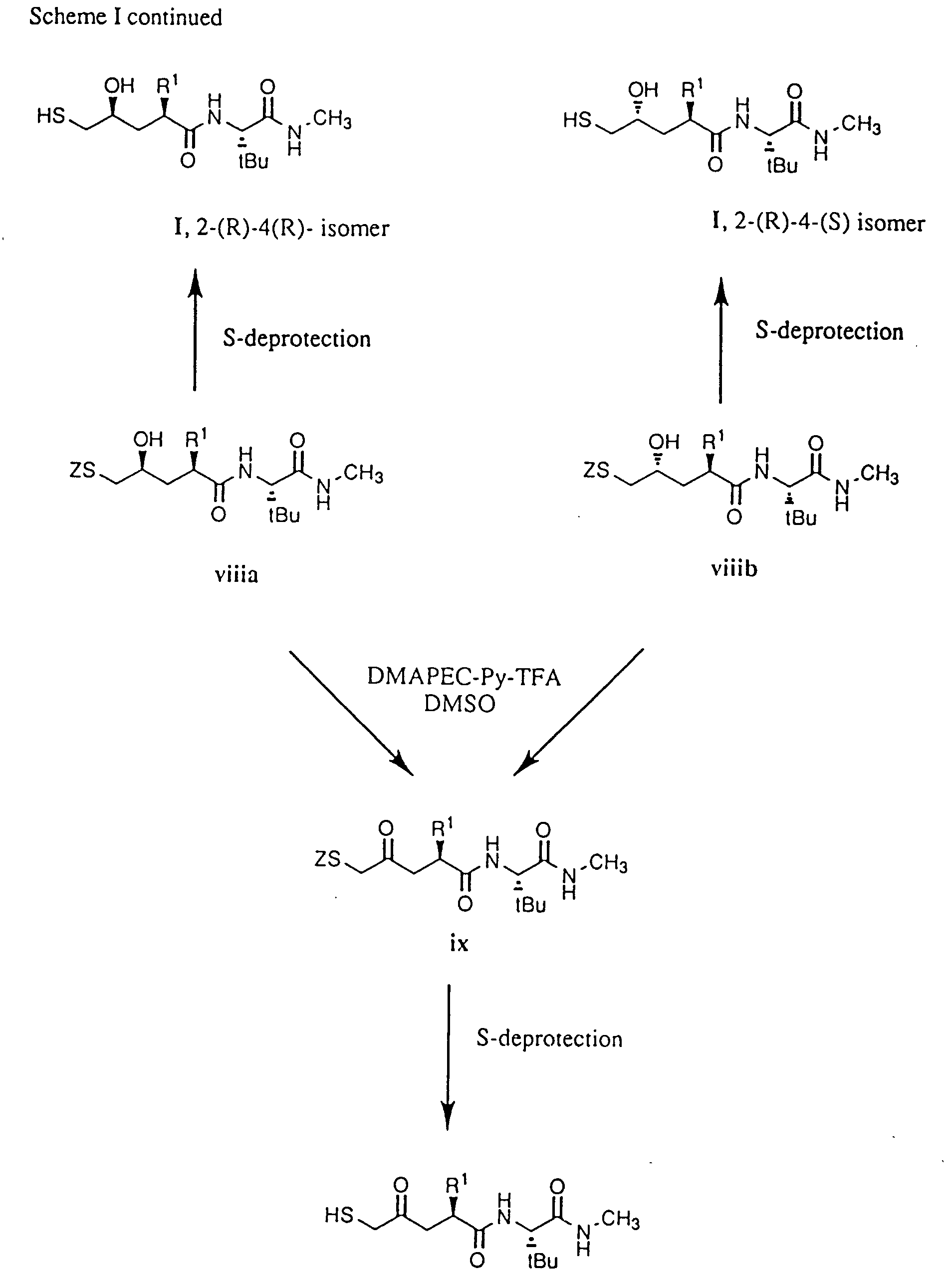

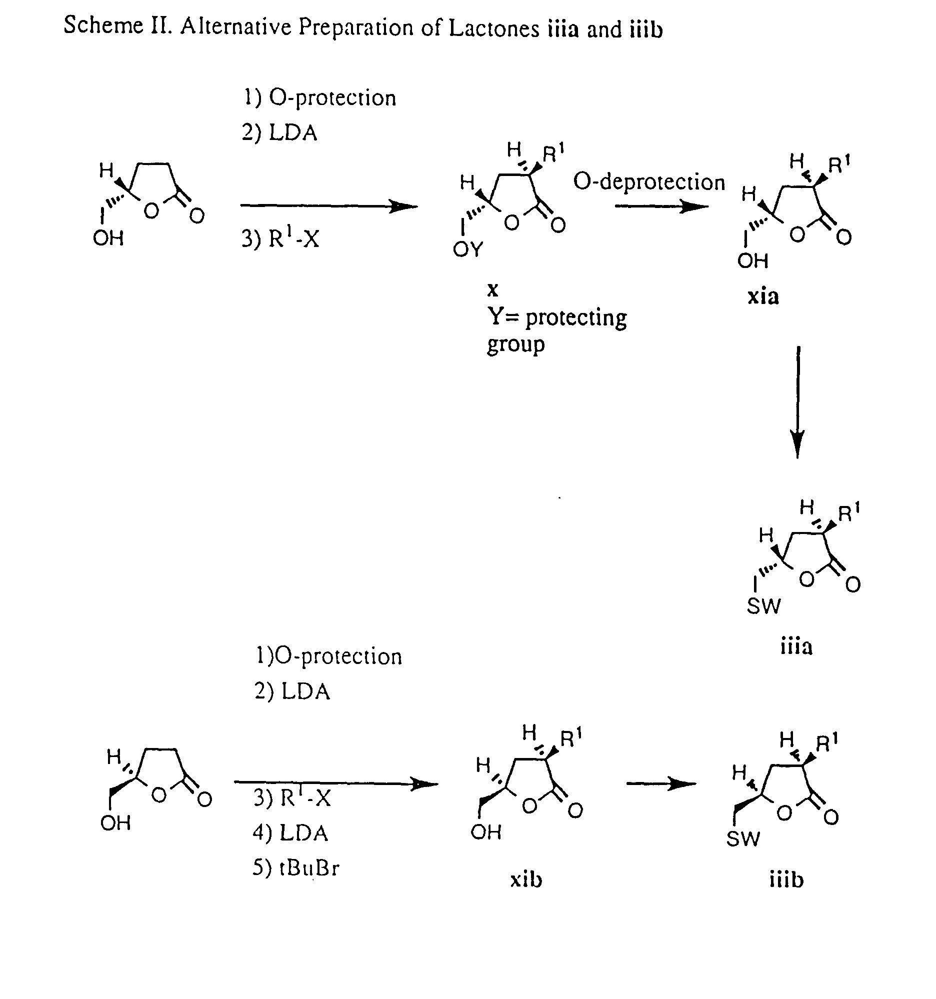

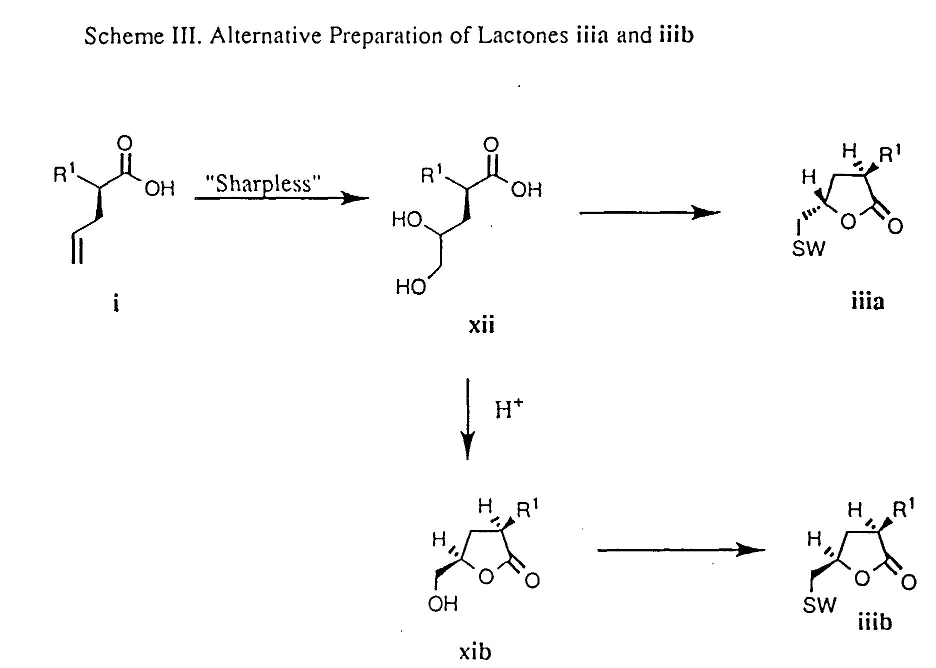

The following examples illustrate the process described above and are included for illustrative purposes only and are not to be construed as limiting to this invention in any way. The chemicals and reagents used in these procedures are either commercially available or readily prepared according to literature procedures by those skilled in the art of synthetic organic chemistry.

### Example 1

### (S)-3-(1-Oxononanyl)-4-(phenylmethyl)-2-oxazolidinone

To a solution of 15.80 g (0.10 mol) of nonanoic acid in 150 mL of dichloromethane at 0°C was added 0.1 mL of N,N-dimethylformamide and 9.59 mL (0.11 mol) of oxalyl chloride. The reaction mixture was allowed to warm to room temperature and stirred overnight. The reaction mixture was then concentrated *in vacuo*, diluted with hexanes and filtered. The filtrate was concentrated in vacuo to provide 17.67g (100%) of nonanoyl chloride which was used without further purification in the next step.

To a solution of 25.2 g (0.142 mol) of (*S*)-(-)-4-benzyl-2-oxazolidinone (Aldrich Chemical Company) in 300 mL of THF cooled to -78°C was added 97.0 mL (0.155 mol) of 1.6M n-butyllithium. The reaction mixture was stirred at -78°C for 1 hour and nonanoyl chloride (17.4 g, 0.129 mol), dissolved in 75 mL of THF, was then added. The resulting mixture was stirred at -78°C for 3 hours and then quenched with 5% HCl solution. The resulting mixture was extracted with ether and the combined organics were washed with water, saturated sodium bicarbonate and brine. The organics were then dried over MgSO₄, filtered and concentrated *in vacuo*. The residue was chromatographed on silica gel, eluting with ethyl acetate/hexanes (1:10), to provide 24.75 g (78%) of the desired product as a white solid.

### Example 2

### (S)-3-(4-Methyl-1-oxopentyl)-4-(phenylmethyl)-2-oxazolidinone

Following the above procedure and substituting 15.0g (0.129 mol) of 4-methylvaleric acid for the nonanoic acid, 23.69g (66%) of the title compound is obtained.

### Example 3

### (4S)-4-Benzyl-3-((2R)-2-heptyl-pent-4-enoyl)oxazolidin-2-one

To a solution of 5.50g (.017 mol) of the oxazolidinone in 40mL of THF, cooled to -78 °C, was added 17.3mL (0.035 mol) of a 2.0M solution of lithium diisopropylamide in heptane/THF/benzene. The resulting mixture was stirred at -78 °C for 1h and then neat allyl bromide (7.5mL, 0.087 mol) was added to the reaction mixture. The reaction was allowed to warm to 0 degrees (ice bath) and stirred for an additional 3h and then quenched with 5% HCI solution. The resulting mixture was extracted with ether and the combined organic layers were washed with water and brine, dried over MgSO₄, filtered and concentrated in vacuo. The residue was chromatographed on silica gel eluting with ethyl acetate/hexanes (1:10) to provide 5.0g (81%) of the desired allylated product as a colorless oil. Electrospray Mass Spec: 358.2 (M+H)⁺.

### Example 4

### (4S)-4-Benzyl-3-((2R)-2-isobutyl-pent-4-enoyl)oxazolidin-2-one

In the same manner as described in Example 3 allylation of the isobutyl substituted oxazolidinone proceeded in 85% yield to provide the product as a colorless oil. CI Mass Spec: 316.3 (M+H).

### Example 5

### (2R)-2-Heptyl-pent-4-enoic acid

To a solution of 8.69g (0.024mol) of the product from Example 3 in 425mL of THF/H20 (3:1), cooled to 0 degrees, was added 10.8mL (0.097mol) of 30% hydrogen peroxide solution followed by 2.03g (0.049mol) of LiOH-H₂O. The resulting solution was stirred at 0 C for 1.5h and then quenched with a solution of 15.4g of sodium sulfite in 100mL of H₂O. The reaction mixture was then acidified to pH 3 with aqueous HCl and extracted with EtOAc. The combined organic layers were washed with water and brine, dried over MgSO₄, filtered and concentrated in vacuo. The residue was chromatographed on silica gel eluting with ethyl acetate/hexanes (1:10) to provide 4.33g (90%) of the desired product as a colorless oil. Cl Mass Spec: 199 (M+H).

### Example 6

### (R)-2-Isobutyl-pent-4-enoic acid

In the same manner as described in Example 5 hydrolysis of the product of Example 4 proceeded in 92% yield to provide the product as a colorless oil. CI Mass Spec: 157.2 (M+H).

### Example 7

### (R)-2-Heptyl-pent-4-enoic acid dimethylamide

To a mixture of 5.00g (0.025mol) of the product of Example 5 and 2.27g (0.028mol) of dimethylamine hydrochloride in DMF at 0° C was added 4.21mL (0.028mol) of diethyl cyanophosphonate followed by 7.38mL (0.053mol) of triethylamine. The reaction mixture was stirred at 0 degrees for 1 and then at room temperature for 3h. The resulting pale yellow suspension was diluted with 750mL of EtOAc/Hex (2:1) and this solution was then washed with 5% aqueous HCl solution, water, saturated sodium bicarbonate and brine. The organic layer was dried over magnesium sulfate, filtered and concentrated in vacuo. The residue was chromatographed on silica gel eluting with ethyl acetate/hexanes (1:4) to provide 5.68g (100%) of the title compound as a colorless oil. CI Mass Spec: 226.3 (M+H).

### Example 8

### (R)-2-Isobutyl-pent-4-enoic acid dimethylamide

In the same manner as described in Example 7 the product of Example 6 gave an 88% yield of the corresponding N,N-dimethyl amide as a colorless oil. CI Mass Spec: 184.2 (M+H).

### Example 9

### (3R,5S)-3-Heptyl-5-iodomethyl-dihydrofuran-2-one

To a mixture of 5.68g (0.025mol) of the product of Example 7 in 90mL of DME/H₂O (1:1) at room temperature was added 7.69g (0.030mol) of iodine. The resulting solution was stirred at room temperature for 60h and then diluted with ether and washed successively with a saturated aqueous solution of Na₂S₂O₃, saturated sodium bicarbonate solution and brine. The organic layer was dried over magnesium sulfate, filtered and concentrated in vacuo. The residue was chromatographed on silica gel eluting with ethyl acetate/hexanes (1:10) to provide 5.11g (63%) of the title compound as a white solid and 0.788g (10%) of the iodo-cis lactone as a colorless oil. CI Mass Spec: 325.3 (M+H).

### Example 10

### (3R, 5S)-5-Iodomethyl-3-isobutyl-dihydrofuran-2-one

In the same manner as described in Example 9 4.14g (0.023mol) of the product of Example 8 gave 4.539g (71%) of the title compound as a colorless oil along with 0.691g (11%) of the corresponding iodo-cis lactone. CI Mass Spec: 283.2 (M+H).

### Example 11

### (3R,5R)-3-Heptyl-5-iodomethyl-dihydrofuran-2-one

To a solution of 7.14g (0.036mol) of the product of Example 5 in 750mL of THF/H₂O (2:1), cooled to 0 degrees, was added 7.22g (0.072mol) of KHCO₃ followed by 11.97g (0.072mol) of KI and then 18.30g (0.072mol) of iodine. The reaction was allowed to warm to room temperature and stirred for 18h. The resulting mixture was diluted with ether and the organic layer was washed with aqueous sodium bisulfite solution, H₂O and brine, dried over MgSO₄, filtered and concentrated in vacuo. The residue was chromatographed on silica gel eluting with ethyl acetate/hexanes (1:10) to provide 8.30g (71%) of the title compound and 3.36g (29%) of the corresponding iodo-trans lactone as white solids. CI Mass Spec: 325.3 (M+H).

### Example 12

### (3R,5R)-5-Iodomethyl-3-isobutyl-dihydrofuran-2-one

In the same manner as described in Example 11 iodolactonization of 6.29g (0.04mol) of the product of Example 4 proceeded to provide 7.21g (63%) of the title compound and 3.77g (33%) of the corresponding iodo-trans lactone as colorless oils. CI Mass Spec: 283.2 (M+H).

### Example 13

### Thioacetic acid S-((2R,4R)-4-heptyl-5-oxo-tetrahydrofuran-2-ylmethyl)ester

To a solution of 6.49g (0.020mol) of the product of Example 11 in 50mL of dry methanol was added 1.19g (0.022mol) of sodium methoxide followed by 5.73mL (0.080mol) of thiolacetic acid. The resulting mixture was heated at reflux for 4.5h and then cooled to room temperature and acidified with 5% HCI solution. The resulting solution was extracted with ether and the combined organic layers were washed with water and brine, dried over MgSO₄, filtered and concentrated in vacuo. The residue was chromatographed on silica gel eluting with ethyl acetate/hexanes (1:10) to provide 5.08g (93%) of colorless oil. CI Mass Spec: 273.3 (M+H).

### Example 14

### Thioacetic acid S-((2S,4R)-4-heptyl-5-oxo-tetrahydrofuran-2-ylmethyl)ester

In the same manner as described in Example 13 the product of Example 9 was converted into the corresponding thioacetate, obtained as a colorless oil in 82% yield. CI Mass Spec: 273.3 (M+H).

### Example 15

### Thioacetic acid S-((4S,2R)-4-heptyl-5-oxo-tetrahydrofuran-2-ylmethyl)ester

In the same manner as described in Example 13 the product of Example 12 was converted into the corresponding thioacetate, obtained as a colorless oil in 75% yield. CI Mass Spec: 231.3 (M+H).

### Example 16

### Thioacetic acid S-((2S,4R)-4-isobutyl-5-oxo-tetrahydrofuran-2-ylmethyl)ester

In the same manner as described in Example 13 the product of Example 10 was converted into the corresponding thioacetate, obtained as a colorless oil in 75% yield. CI Mass Spec: 231.3 (M+H).

### Example 17

### (3R,5R)-3-Heptyl-5-tritylsulfanylmethyl-dihydrofuran-2-one

To a solution of the product of Example 13 in 100mL of methanol, cooled to 0 °C, was added 2.69g (0.071mol) of solid sodium borohydride in portions over 15 minutes. The reaction was then concentrated in vacuo, acidified with 10% HCI solution and extracted with methylene chloride. The combined organic layers were washed with water and brine, dried over MgSO₄, filtered and concentrated in vacuo. The resulting crude thiol was dissolved in 50mL of trifluoroacetic acid and 5.98g (0.023mol) of trityl alcohol was added. After stirring at room temperature for 1h the reaction mixture was concentrated in vacuo and the residue was chromatographed on silica gel eluting with ethyl acetate/hexanes (1:50) to provide 5.26g (61%) of the desired product as a colorless oil. CI Mass Spec: 473.5 (M+H).

### Example 18

### (3R,5S)-3-Heptyl-5-tritylsulfanylmethyl-dihydrofuran-2-one

In the same manner as described in Example 17 the product of Example 14 was converted into the corresponding S-trityl derivative, obtained as a colorless oil in 65% yield. CI Mass Spec: 473.5 (M+H).

### Example 19

### (3R,5R)-3-Isobutyl-5-tritylsulfanylmethyl-dihydrofuran-2-one

In the same manner as described in Example 17 the product of Example 15 was converted into the corresponding S-trityl derivative, obtained as a colorless oil in 64% yield. CI Mass Spec: 431.4 (M+H).

### Example 20

### (3R,5S)-3-Isobutyl-5-tritylsulfanylmethyl-dihydrofuran-2-one

In the same manner as described in Example 17 the product of Example 16 was converted into the corresponding S-trityl derivative, obtained as a colorless oil in 61% yield. CI Mass Spec: 431.5 (M+H).

### Example 21

### (2R)-2-((2R)-2-Hydroxy-3-tritylsulfanylpropyl)nonanoic acid

To a solution of 3.72g (7.88mmol) of the product of Example 17 in 100mL of methanol/THF (1:1) at room temperature was added 16.7mL of 1.0N NaOH solution. The reaction was stirred at room temperature for 1h and then diluted with 100mL of H₂O and carefully acidified to pH 6 with 5% HCl solution. The resulting mixture was extracted with EtOAc and the combined organic layers were washed with water and brine, dried over MgSO₄, filtered and concentrated in vacuo. The residue, 3.72g (100%) of the crude hydroxy-acid was used in the next step (Example 25) without further purification. Electrospray Mass Spec: 489.4 (M-H)⁻.

### Example 22

### (2R)-2-((2S)-2-Hydroxy-3-tritylsulfanylpropyl)nonanoic acid

In the same manner as described in Example 21 the product of Example 18 was converted into the corresponding hydroxy-acid derivative, obtained as a colorless oil in 95% yield. Electrospray Mass Spec: 489.4 (M-H)⁻.

### Example 23

### (2R,4R)-4-Hydroxy-2-isobutyl-5-tritylsulfanylpentanoic acid

In the same manner as described in Example 21 the product of Example 19 was converted into the corresponding hydroxy-acid derivative, obtained as a colorless oil in 99% yield. Electrospray Mass Spec: 447.3 (M-H)⁻.

### Example 24

### (2R,4S)-4-Hydroxy-2-isobutyl-5-tritylsulfanylpentanoic acid

In the same manner as described in Example 21 the product of Example 20 was converted into the corresponding hydroxy-acid derivative, obtained as a colorless oil in 99% yield. Electrospray Mass Spec: 447.3 (M-H)⁻.

### Example 25

### (2R)-2-[(2R)-2-(tert-Butyl-dimethylsilanyloxy)-3-tritylsulfanylpropyl]nonanoic acid

To a solution of 3.72g (7.88mmol) of the crude product of Example 21 dissolved in 10mL of DMF was added 2.68g (0.039mol) of imidazole followed by 2.85g (0.019mol) of t-butyldimethylsilyl chloride. The reaction mixture was stirred at room temperature for 2h and then poured into 200mL of H₂O. The resulting solution was extracted with ether and the combined organic layers were washed with water and brine, dried over MgSO₄, filtered and concentrated in vacuo. The residue was dissolved in 10mL of methanol/THF (1:1) and 5.0mL of 1N NaOH solution was added. After stirring for 0.75h at room temperature the reaction mixture was acidified with 5% HCI solution to pH 5 and then extracted with ether. The combined organic layers were washed with water and brine, dried over MgSO₄, filtered and concentrated in vacuo. The residue was chromatographed on silica gel eluting with ethyl acetate/hexanes (1:10) to provide 4.76g (100%) of the desired product as a colorless oil. Electrospray Mass Spec: 603.5 (M-H)⁻.

### Example 26

### (2R)-2-[(2S)-2-(tert-Butyl-dimethylsilanyloxy)-3-tritylsulfanylpropyl]nonanoic acid

In the same manner as described in Example 25 the product of Example 22 was converted into the corresponding TBDMS ether-acid derivative, obtained as a colorless oil in 83% yield. Electrospray Mass Spec: 603.5 (M-H)⁻.

### Example 27

### (2R,4S)-4-(tert-Butyldimethylsilanyloxy)-2-isobutyl-5-tritylsulfanylpentanoic acid

In the same manner as described in Example 25 the product of Example 24 was converted into the corresponding TBDMS ether-acid derivative, obtained as a colorless oil in 91% yield. Electrospray Mass Spec: 561.4 (M-H)⁻.

### Example 28

### (2R,4R)-4-(tert-Butyldimethylsilanyloxy)-2-isobutyl-5-tritylsulfanylpentanoic acid

In the same manner as described in Example 25 the product of Example 23 was converted into the corresponding TBDMS ether-acid derivative, obtained as a colorless oil in 95% yield. Electrospray Mass Spec: 561.4 (M-H)⁻.

### Example 29

### (2R)-2-[(2R)-2-(tert-Butyl-dimethylsilanyloxy)-3-tritylsulfanylpropyl]-nonanoic acid ((1S)-2,2-dimethyl-1-methylcarbamylpropyl)amide

To a solution of 4.76g (7.88mmol) of the product of Example 25 dissolved in 150mL of dichloromethane was added 1.42g (9.85mmol) of t-butyl glycine-N-methylamide followed by 1.86mL (0.013mmol) of triethylamine and 1.67mL (0.011mmol) of diethylcyanophosphonate. The reaction mixture was stirred at room temperature for 12h and then concentrated in vacuo. The resulting residue was diluted with ether and the organics were washed with 5% HCl solution, water and brine. The organic layer was then dried over MgSO₄, filtered and concentrated in vacuo to provide 5.13g (89%) of the desired product as an oil pure enough for use in the next step. FAB Mass Spec: 753.4 (M+Na).

### Example 30

### (2R)-2-[(2S)-2-(tert-Butyl-dimethylsilanyloxy)-3-tritylsulfanylpropyl]-nonanoic acid ((1S)-2,2-dimethyl-1-methylcarbamylpropyl)amide

Using the same procedure described in Example 29 6.80g (11.26mmol) of the product of Example 26 produced 7.71g (94%) of the desired product as a colorless oil. FAB Mass Spec: 753.4 (M+Na).

### Example 31

### (2R,4R)-4-(tertButyldimethylsilanyloxy)-2-isobutyl-5-tritylsulfanylpentanoic acid ((1S)-2,2-dimethyl-1-methylcarbamoylpropyl)amide

Using the same procedure described in Example 29 5.81g (10.33mmol) of the product of Example 28 produced 4.62g (65%) of the desired product as a colorless oil. ¹H NMR (300MHz, CDCl₃) δ 7.44 (m, 5H), 7.26 (m, 10H), 6.16 (m, 1H), 5.96 (d, J=9Hz, 1H), 4.19 (d, J=9Hz, 1H), 3.58 (m, 1H), 2.76 (d, J=4.7Hz, 3H), 2.29 (dd, J=6.2,12Hz, 1H), 2.13 (dd, J=4.5,12Hz, 1H), 2.0 (m, 1H), 1.7-1.1 (m, 3H), 0.92 (s, 9H), 0.85 (s, 9H), 0.82 (dd, J=6,10Hz), -0.039 (s, 3H), -0.078 (s, 3H).

### Example 32

### (2R,4S)-4-(tertButyldimethylsilanyloxy)-2-isobutyl-5-tritylsulfanylpentanoic acid ((1S)-2,2-dimethyl-1-methylcarbamoylpropyl)amide

Using the same procedure described in Example 29 7.20g (12.81mmol) of the product of Example 27 produced 5.53g (63%) of the desired product as a colorless oil. Electrospray Mass Spec: 711.4 (M+Na)⁺.

### Example 33

### (2R)-2-((2R)-2-Hydroxy-3-tritylsulfanylpropyl)nonanoic acid ((1S)-2,2-dimethyl-1-methylcarbamoylpropyl)amide

To a solution of 5.13g (7.027mmol) of the product of Example 29 dissolved in 75mL of THF was added 17.6mL (0.018mmol) of a 1.0M solution of tetrabutylammonium fluoride in THF. The reaction mixture was stirred at room temperature for 4h and then diluted with ether. The resulting solution was washed with water and brine, dried over MgSO₄, filtered and concentrated in vacuo. The residue was chromatographed on silica gel eluting with EtOAc/Hexanes (gradient: 1:3-1:1) to provide 2.78g (64%) of the desired product as a colorless oil. Electrospray Mass Spec: 617.5 (M+H)⁺.

### Example 34

### (2R)-2-((2S)-2-Hydroxy-3-tritylsulfanylpropyl)nonanoic acid ((1S)-2,2-dimethyl-1-methylcarbamoylpropyl)amide

Using the same procedure described in Example 33 7.63g (0.126mmol) of the product of Example 30 produced 4.22g (66%) of the desired product as a colorless oil. Electrospray Mass Spec: 617.5 (M+H)⁺.

### Example 35

### (2R,4R)-4-Hydroxy-2-isobutyl-5-tritylsulfanylpentanoic acid ((1S)-2,2-dimethyl-1-methylcarbamoylpropyl)amide

Using the same procedure described in Example 33 4.58g (0.126mmol) of the product of Example 31 produced 3.66g (96%) of the desired product as a colorless oil. Electrospray Mass Spec: 575.4 (M+H)⁺.

### Example 36

### (2R,4S)-4-Hydroxy-2-isobutyl-5-tritylsulfanylpentanoic acid ((1S)-2,2-dimethyl-1-methylcarbamoylpropyl)amide

Using the same procedure described in Example 31 5.50g (0.126mmol) of the product of Example 30 produced 3.87g (84%) of the desired product as a colorless oil. Electrospray Mass Spec: 575.3 (M+H)⁺.

### Example 37

### (2R)-2-((2R)-2-Hydroxy-3-mercaptopropyl)nonanoic acid ((1S)-2,2-dimethyl-1-methylcarbamoylpropyl)amide

To a solution of 0.700g (1.136mmol) of the product of Example 33 and 0.363mL (2.273mmol) of triethylsilane dissolved in 10mL of dichloromethane was dropwise added 10mL of trifluoroacetic acid. The reaction mixture was stirred at room temperature for 0.5h and then concentrated in vacuo. The residue was chromatographed on silica gel eluting with EtOAc/Hexanes (gradient: 1:3-1:1) to provide 0.263g (62%) of the desired product as a colorless oil. Electrospray Mass Spec: 375.4 (M+H)⁺.

### Example 38

### (2R)-2-((2S)-2-Hydroxy-3-mercaptopropyl)nonanoic acid ((1S)-2,2-dimethyl-1-methylcarbamoylpropyl)amide

Using the same procedure described in Example 37 1.0g (1.62mmol) of the product of Example 34 produced 0.24g (40%) of the desired product as a colorless oil. Electrospray Mass Spec: 375.4 (M+H)⁺.

### Example 39

### (2R,4R)-4-Hydroxy-2-isobutyl-5-mercaptopentanoic acid ((1S)-2,2-dimethyl-1-methylcarbamoylpropyl)amide

Using the same procedure described in Example 37 1.50g (2.61mmol) of the product of Example 35 produced 0.47g (54%) of the desired product as a colorless oil. Electrospray Mass Spec: 333.3 (M+H)⁺.

### Example 40

### (2R,4S)-4-Hydroxy-2-isobutyl-5-mercaptopentanoic acid ((1S)-2,2-dimethyl-1-methylcarbamoylpropyl)amide

Using the same procedure described in Example 37 1.50g (2.61mmol) of the product of Example 36 produced 0.14g (16%) of the desired product as a colorless oil. Electrospray Mass Spec: 333.3 (M+H)⁺.

### Example 41

### (2R)-2-(2-Oxo-3-tritylsulfanylpropyl)nonanoic acid ((1S)-2,2-dimethyl-1-methylcarbamoylpropyl)amide

To a solution of 0.962g (1.562mmol) of the product of Example 33 in 3mL of DMSO was added 0.505mL (6.246mmol) of pyridine followed by 0.120mL (1.562mmol) of trifluoroacetic acid and 0.898 (4.685mmol) of 1-(3-dimethylaminopropyl)-3-ethylcarbodiimide hydrochloride. The reaction mixture was stirred at room temperature for 36h and then diluted with 200mL of EtOAc. The resulting solution was washed with 0.1N HCI solution, water, saturated NaHCO₃ solution and brine, dried over MgSO₄, filtered and concentrated in vacuo. The residue, 0.948g (99%) was used in the next step without purification. Electrospray Mass Spec: 615.4 (M+H)⁺.

### Example 42

### (2R)-2-Isobutyl-4-oxo-5-tritylsulfanylpentanoic acid ((1S)-2,2-dimethyl-1-methylcarbamoylpropyl)amide

Using the same procedure described in Example 41 1.25g (2.18mmol) of the product of Example 35 produced 1.25g (100%) of the desired product as a colorless oil. Electrospray Mass Spec: 573.4 (M+H)⁺.

### Example 43

### (2R)-2-(3-Mercapto-2-oxopropyl)nonanoic acid ((1S)-2,2-dimethyl-1-methylcarbamoylpropyl)amide

Using the same procedure described in Example 37 1.985g (3.23mmol) of the product of Example 41 produced 0.506g (42%) of the desired product as a colorless oil. Electrospray Mass Spec: 373.4 (M+H)⁺.

### Example 44

### (2R)-2-Isobutyl-5-mercapto-4-oxopentanoic acid ((1S)-2,2-dimethyl-1-methylcarbamoylpropyl)amide

Using the same procedure described in Example 37 2.49g (4.35mmol) of the product of Example 42 produced 0.462g (32%) of the desired product as a colorless oil. Electrospray Mass Spec: 331.3 (M+H)⁺.

### Pharmacology

Table 1 summarizes the pharmacological data obtained for the four diastereomeric alcohols and two mercaptoketones of this invention where R is isobutyl or heptyl. The pharmacological procedures follow the table. The data show that the 2(*S*) hydroxy diastereomers have superior and **unexpected** potency over the 2(*R*) hydroxy analogs both *in vivo* and *in vitro,* the latter being less potent than the keto compounds.

**Table 1.**

| In vitro and In vivo Inhibition of Matrix Metalloproteinases | | | | | | |
|---|---|---|---|---|---|---|
| Example | R² & R⁶ | R¹ | Collagenase IC₅₀ (nM) or % inhibition | Stromelysin IC₅₀ (nM) or % inhibition | Gelatinase IC₅₀ (nM) | in vivo % gelatinase inhibition |
| 43 | =O | heptyl | 30.0 | 28 | | 0.73/50mpk* |
| 44 | =O | isobutyl | 38.0 | 58% @1µM | | 4.8/60mpk |
| 38 | 2(*S*) OH | heptyl | 30.0 | 96%@0.1µM | | 22.2/50mpk |
| 40 | 2(*S*) OH | isobutyl | 41.0 | 51%@1µM | 19 | 84.8/50mpk |
| 35 | 2(*R*) OH | heptyl | 72%@1µM | 38%@1µM | | 0.44/50mpk |
| 39 | 2(*R*) OH | isobutyl | 228 | 6%@300nM | 106 | 20.4/50 mpk |

| | | | | | | |
|---|---|---|---|---|---|---|
| *mpk= milligrams/kilogram | | | | | | |

### Stromelysin Inhibition in vitro.

The assay is based on the cleavage of the thiopeptide substrate ((Ac-Pro-Leu-Gly(2-mercapto-4-methylpentanoyl)Leu-Gly-OEt), Bachem Bioscience) by the enzyme stromelysin, releasing the substrate product which forms a colorimetric reaction with DTNB ((5,5'-dithiobis(2-nitrobenzoic acid)). The thiopeptide substrate is made up as a 20mM stock in 100% DMSO and stored frozen while the DTNB stock is dissolved in 100% DMSO and stored as a 100mM stock at room temperature. Both the substrate and DTNB are diluted to 1mM with assay buffer (50mM HEPES, pH 7.0, 5 mM CaCl₂) before use. The stock of human recombinant stromelysin (truncated, Affymax) is diluted with assay buffer to a final concentration of 12.5 nM in the reaction.

The enzyme, DTNB, and substrate (10µM final concentration) with/without inhibitor (total reaction volume of 200µL) is added to a 96 well plate and then the increase in color is monitored spectrophotometrically for 5 minutes at 405 nanometers (nm) on a plate reader. The increase in OD₄₀₅ is plotted and the slope of the line is calculated which represents the reaction rate.

The linearity of the reaction rate is confirmed (r²>0.85). The mean of the control rate is calculated and compared for statistical significance (p<0.05) with drug-treated rates using Dunnett's multiple comparison test. Dose-response relationships can be generated using multiple doses of drug and IC₅₀ values with 95% Cl are estimated using linear regression (IPRED, HTB) (Weingarten and Feder, Spectrophometric Assay For Vertebrate Collagenase, *Anal. Biochem* **147**, 437-440 (1985)).

### Gelatinase Inhibition in vitro.

The assay is based on the cleavage of the thiopeptide substrate ((Ac-Pro-Leu-Gly(2-mercapto-4-methylpentanoyl)Leu-Gly-OEt), Bachem Bioscience) by the enzyme gelatinase, releasing the substrate product which forms a colorimetric reaction with DTNB ((5,5'-dithiobis(2-nitrobenzoic acid)). The thiopeptide substrate is made up fresh as a 20mM stock in 100% DMSO and the DTNB is dissolved in 100% DMSO and stored in dark as a 100mM stock at room temperature. Both the substrate and DTNB are diluted to 1mM with assay buffer (50mM HEPES, pH 7.0, 5 mM CaCl₂, 0.2% Brij) before use. The stock of human neutrophil gelatinase B is diluted with assay buffer to a final concentration of 0.15 nM.

The assay buffer, enzyme, DTNB, and substrate (500µM final concentration) with/without inhibitor (total reaction volume of 200µL) is added to a 96 well plate and then the increase in color is monitored spectrophotometrically for 5 minutes at 405 nanometers (nm) on a plate reader. The increase in OD₄₀₅ is plotted and the slope of the line is calculated which represents the reaction rate.

The linearity of the reaction rate is confirmed (r²>0.85). The mean of the control rate is calculated and compared for statistical significance (p<0.05) with drug-treated rates using Dunnett's multiple comparison test. Dose-response relationships can be generated using multiple doses of drug and IC₅₀ values with 95% CI are estimated using linear regression (IPRED, HTB). (Weingarten and Feder, Spectrophometric Assay For Vertebrate Collagenase, *Anal*. *Biochem* **147**, 437-440 (1985)).

### Collagenase Inhibition in vitro

The assay is based on the cleavage of a peptide substrate ((Dnp-Pro-Cha-Gly-Cys(Me)-His-Ala-Lys(NMa)-NH₂), Peptide International, Inc.) by collagenase releasing the fluorescent NMa group which is quantitated on the fluorometer. Dnp quenches the NMa fluorescence in the intact substrate. The assay is run in HCBC assay buffer (50mM HEPES, pH 7.0, 5mM Ca⁺², 0.02% Brij, 0.5% cysteine), with human recombinant fibroblast collagenase (truncated, mw 18,828, WAR, Radnor). Substrate is dissolved in methanol and stored frozen in 1mM aliquots. Collagenase is stored frozen in buffer in 25 µM aliquots. For the assay, substrate is dissolved in HCBC buffer to a final concentration of 10 µM and collagenase to a final concentration of 5nM. Compounds are dissolved in methanol, DMSO, or HCBC. The methanol and DMSO are diluted in HCBC to <1%. Compounds are added to the 96 well plate containing enzyme and the reaction is started by the addition of substrate.

The reaction is read (excitation 340 nm, emission 444nm) for 10 minutes and the increase in fluorescence over time is plotted as a linear line. The slope of the line is calculated and represents the reaction rate.

The linearity of the reaction rate is confirmed (r²>0.85). The mean of the control rate is calculated and compared for statistical significance (p<0.05) with drug-treated rates using Dunnett's multiple comparison test. Dose-response relationships can be generated using multiple doses of drug and IC₅₀ values with 95% CI are estimated using linear regression (IPRED, HTB) (Bickett, D. M. et al., A High Throughput Fluorogenic Substrate For Interstitial Collagenase (MMP-1) and Gelatinase (MMP-9), *Anal*. *Biochem*. **212,** 58-64 (1993)).

### In vivo MMP Inhibition

A 2 cm piece of dialysis tubing (molecular weight cut-off 12-14,000, 10 mm flat width) containing matrix metalloproteinase enzyme (stromelysin, collagenase or gelatinase in 0.5 mL of buffer) is implanted either ip or sc (in the back) of a rat (Sprague-Dawley, 150-200g) or mouse (CD-1, 25-50g) under anesthesia. Drugs are administered PO, IP, SC or IV through a canula in the jugular vein. Drugs are administered in a dose volume of 0.1 to 0.25 mL/animal. Contents of the dialysis tubing is collected and enzyme activity assayed.

Enzyme reaction rates for each dialysis tube are calculated. Tubes from at least 3 different animals are used to calculate the mean± sem. Statistical significance (p<0.05) of vehicle-treated animals versus drug-treated animals is determined by analysis of variance. (*Agents and Actions* 21: 331, 1987)

### Pharmaceutical Composition

Compounds of this invention may be administered neat or with a pharmaceutically acceptable carrier to a patient in need thereof. The pharmaceutically acceptable carrier may be solid or liquid.

Applicable solid carriers can include one or more substances which may also act as flavoring agents, lubricants, solubilizers, suspending agents, fillers, glidants, compression aids, binders or tablet-disintegrating agents or an encapsulating material. In powders, the carrier may be a finely divided solid which is in admixture with the finely divided active ingredient. In tablets, the active ingredient may be mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such a solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above, e.g., cellulose derivatives, preferable sodium carboxymethyl cellulose solution), alcohols (including monohydric alcohols and polyhydric alcohols, e.g., glycols) and their derivatives, and oils (e.g., fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be either liquid or solid composition form.

The dosage to be used in inhibiting the matrix metalloproteinases in a patient must be subjectively determined by the attending physician. The variables involved include the severity of the dysfunction, and the size, age, and response pattern of the patient. Treatment will generally be initiated with small dosages less than the optimum dose of the compound. Thereafter the dosage is increased until the optimum effect under the circumstances is reached. Precise dosages will be determined by the administering physician based on experience with the individual subject treated and standard medical principles.

Preferably the pharmaceutical composition is in unit dosage form, e.g., as tablets or capsules. In such form, the composition is sub-divided in unit dose containing appropriate quantities of the active ingredient; the unit dosage form can be packaged compositions, for example packed powders, vials, ampoules, prefilled syringes or sachets containing liquids. The unit dosage form can be, for example, a capsule or tablet itself, or it can be the appropriate number of any such compositions in package form.

## Claims

1. A diastereomerically and enantiomerically pure compound according to the formula: 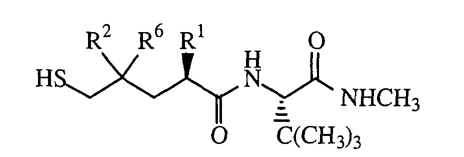
wherein R¹ is C₁-C₁₂ alkyl, straight or branched and optionally substituted by halogen, hydroxy, C₁-C₆ alkoxy, amino, carboxyl, C₁-C₆ alkoxycarbonyl, carboxamido, nitrile, mono- or di-(C₁-C₆)alkylamino, thio, C₁-C₆ alkylthio, aryl, -Oaryl or -OCH₂aryl where aryl is optionally substituted with C₁-C₆ alkyl, C₁-C₆ alkoxy, carboxy, halogen, cyano, nitro, carboxamido, hydroxy or C₁-C₆ alkanesulfonyl and aryl is a 5 to 10 membered carbocyclic or heterocyclic mono or bicyclic aromatic group;
and R² is α-OH or β-OH and R⁶ is H or R² and R⁶ together form carbonyl.

2. A diasteromerically and enantiomerically pure compound according to the formula: 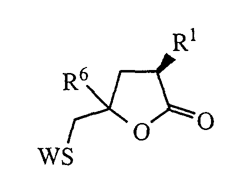
where R¹ is as defined in Claim 1;
W is a thiol protecting group and
R⁶ is α-H or β-H.

3. A compound according to claim 2 where W is acetyl or triphenylmethyl.

4. A diasteromerically and enantiomerically pure compound according to the formula: 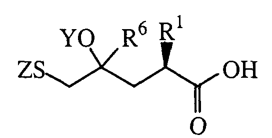
wherein Y is H or a hydroxyl protecting group;
Z is H or a thiol protecting group;
R¹ is as defined in Claim 1 and
R⁶ is α-H or β-H.

5. A diastereomerically and enantiomerically pure compound according to the formula: 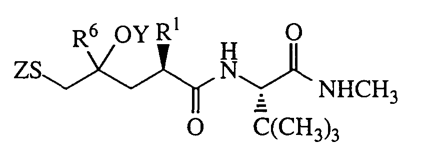
wherein Y is H or a hydroxyl protecting group;
Z is H or a thiol protecting group;
R¹ is as defined in Claim 1 and
R⁶ is α-H or β-H.

6. A compound according to Claim 4 or Claim 5 wherein Y is tert-butyldimethylsilyl and Z is trityl.

7. A compound according to Claim 4 or Claim 5 wherein Y is H and Z is trityl.

8. A diastereomerically and enantiomerically pure compound according to the formula: 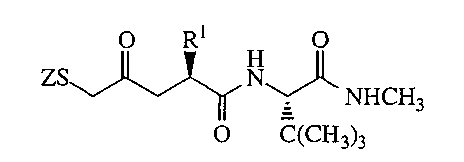
where Z is as defined in Claim 5 with the proviso that Z is not H;
and R¹ is as defined in Claim 1.

9. A compound according to claim 8 where Z is trityl.

10. A compound according to any one of claims 1 to 9 wherein R¹ is heptyl or isobutyl.

11. A process for the preparation of a diastereomerically and enantiomerically pure compound of formula 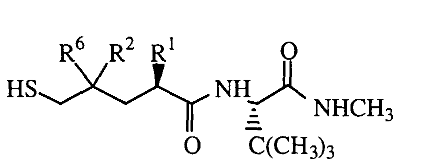 according to claim 1, wherein R¹ is as defined in Claim 1; R² is (*S*)-OH and R⁶ is H,
which comprises deprotecting a compound **viiia** 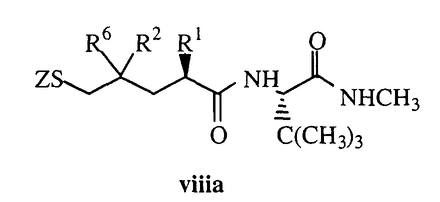 wherein Z is a thiol protecting group.

12. A process for the preparation of a diastereomerically and enantiomerically pure compound of formula 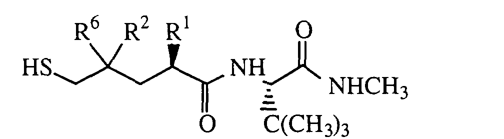 according to claim 1 where R¹ is as defined in Claim 1; R² is (*S*)-OH and R⁶ is H, which comprises:
(1) reacting a *(3R,5S)*-5-iodomethyl-3-R¹-dihydrofuran-2-one with the anion of HSW, where W is a thiol protecting group to provide lactone **iiia**, 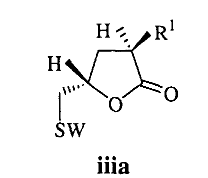
(2) deprotection of the sulfur of lactone **iiia** of step (1) under reducing conditions, acid hydrolysis or base hydrolysis to provide compound **iva'**, 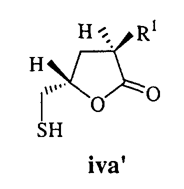
(3) protection of the free thiol of step (2) above to provide compound **iva** where Z is a thiol protecting group, 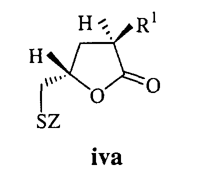
(4) hydrolysis of the furanone of step (3) with aqueous acid or base to give compound **va,** 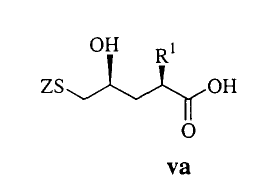 a thiol-protected (2R)-2-R¹-(4R)-4-hydroxy-5-mercaptopentanoic acid, where Z is as defined above,
(5) protection of the hydroxy group of compound **va** of step (4) with a protecting group Y to give compound **via**, 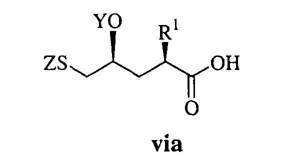 where Y is a hydroxyl protecting group,
(6) amidation of carboxylic acid **via** of step (5) with (2S)-t-butylglycine N-methylamide and a peptide coupling reagent, to provide compound **viia,** 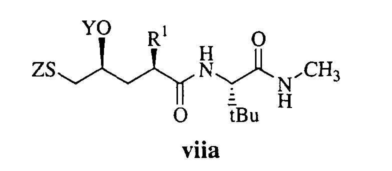 where Y and Z are as defined above,
(7) removal of the protecting group Y from the hydroxyl group of compound **viia** of step (6) to provide alcohol **viiia,** 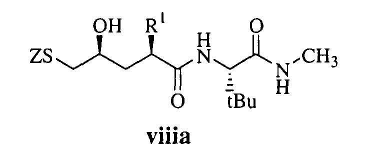 where Z is as defined above,
(8) removal of the thiol protecting group of compound **viiia** of step (7) to provide a compound of the above formula.

13. The process according to claim 12 for the preparation of a diastereomerically and enantiomerically pure compound of formula 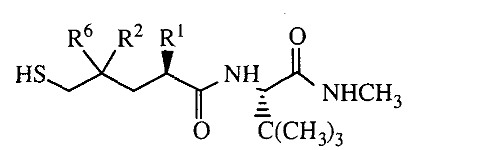 according to claim 1, where R¹ is as defined in Claim 1; R² is (*S*)-OH and R⁶ is H, which comprises:
(1) reacting a *(3R,5S)*-5-iodomethyl-3-R¹-dihydrofuran-2-one with the sodium, lithium or potassium salt of thiolacetic acid, to provide lactone **iiia** where W is acetyl, 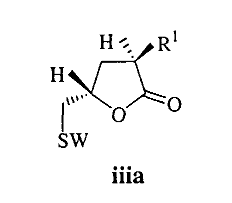
(2) deprotection of the sulfur of lactone **iiia** of step (1) wherein the acetyl group is removed by treatment with sodium borohydride in methanol or ethanol to provide compound **iva'**, 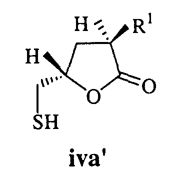
(3) protection of the free thiol of step (2) above to provide compound **iva** where Z is triphenylmethyl, 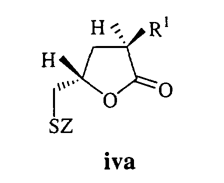
(4) hydrolysis of the furanone of step (3) with aqueous acid or base, preferably sodium hydroxide, to give compound **va**, a thiol-protected (2R)-2-R¹-(4R)-4-hydroxy-5-mercaptopentanoic acid, where Z is triphenylmethyl, 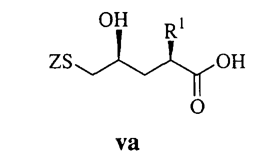
(5) protection of the hydroxy group of compound **va** of step (4) with the t-butyldimethylsilyl protecting group to give compound **via** where Y is t-butyldimethylsilyl, 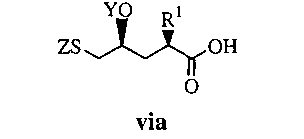
(6) amidation of carboxylic acid **via** of step (5) with (2S)-t-butylglycine N methylamide and the peptide coupling reagent diethylcyanophosphonate, and triethylamine, to provide compound **viia** where Y is t-butyldimethylsilyl and Z is triphenylmethyl, 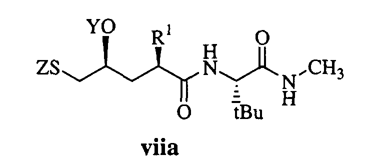
(7) removal of the t-butyldimethylsilyl protecting group Y from the hydroxyl group of compound **viia** of step (6) with tetrabutylammonium fluoride to provide alcohol **viiia,** where Z is triphenylmethyl, 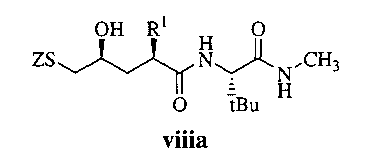
(8) removal of the triphenylmethyl thiol protecting group of compound **viiia** of step (7) with trifluoroacetic acid and triethylsilane to provide a compound of the above formula.

14. A process for the preparation of a compound of formula 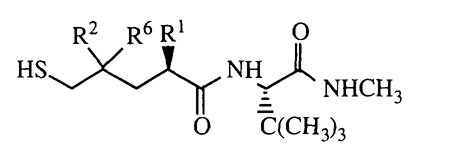 according to claim 1, wherein R¹ is as defined in Claim 1; R² is (*R*)-OH and R⁶ is H
which comprises deprotecting a compound **viiib** 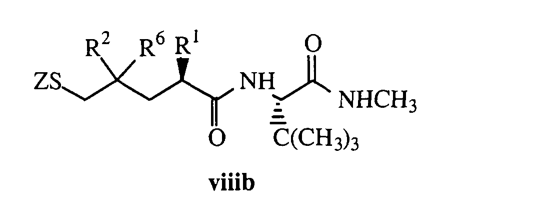 wherein Z is a thiol protecting group.

15. A process for the preparation of a compound of formula 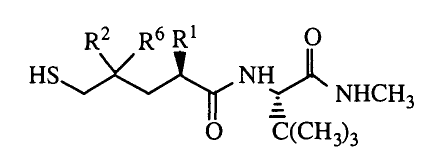 according to claim 1, where R¹ is as defined in Claim 1; R² is (*R*)-OH and R⁶ is H which comprises:
(1) reacting a *(3R,5R*)-5-iodomethyl-3-R¹-dihydrofuran-2-one with the anion of HSW, where W is a thiol protecting group to provide lactone **iiib,** 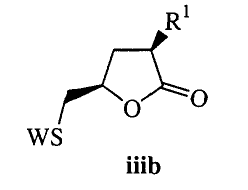
(2) deprotection of the sulfur of lactone **iiib** of step (1) under reducing conditions, acid hydrolysis or base hydrolysis to provide compound **ivb',** 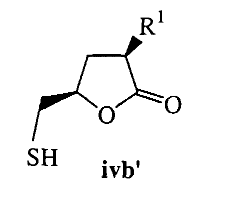
(3) protection of the free thiol of step (2) above to provide compound **ivb** where Z is a thiol protecting group, 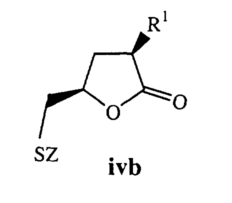
(4) hydrolysis of the furanone of step (3) with aqueous acid or base to give compound **vb,** a thiol-protected (2R)-2-R¹-(4R)-4-hydroxy-5-mercaptopentanoic acid,
where Z is as defined above, 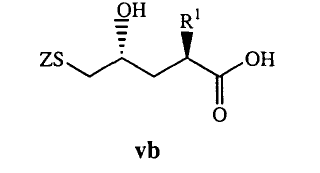
(5) protection of the hydroxy group of compound **vb** of step (4) with a protecting group Y to give compound **vib** where Y is a hydroxyl protecting group, 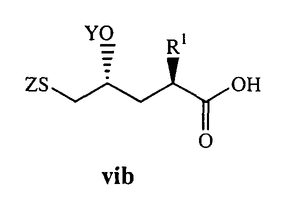
(6) amidation of carboxylic acid **vib** of step (5) with (2S)-t-butylglycine N-methylamide and a peptide coupling reagent, to provide compound **viib** where Y and Z are as defined above, 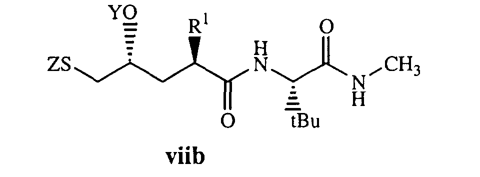
(7) removal of the protecting group Y from the hydroxyl group of compound **viib** of step (6) to provide alcohol **viiib,** where Z is as defined above, 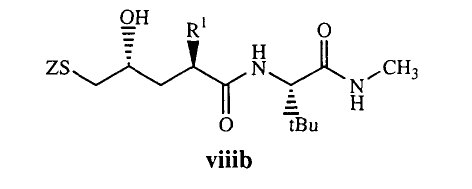
(8) removal of the thiol protecting group of compound **viiib** of step (7) to provide a compound of the above formula.

16. The process according to claim 15 for the preparation of a diastereomerically and enantiomerically pure compound of formula 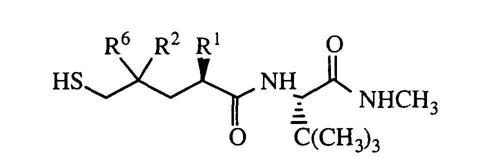 according to claim 1, where R¹ is as defined in Claim 1; R² is (*R*)-OH and R⁶ is H, which comprises:
(1) reacting a *(3R,5R)*-5-iodomethyl-3-R¹-dihydrofuran-2-one with the sodium, lithium or potassium salt of thiolacetic acid, to provide lactone **iiib** where W is acetyl, 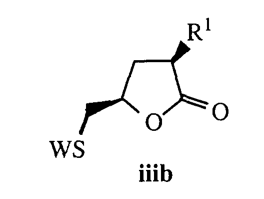
(2) deprotection of the sulfur of lactone **iiib** of step (1) wherein the acetyl group is removed by treatment with sodium borohydride in methanol or ethanol to provide compound **ivb'**, 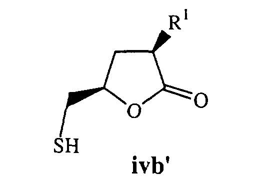
(3) protection of the free thiol of step (2) above to provide compound **ivb** where Z is triphenylmethyl, 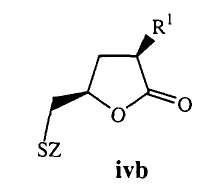
(4) hydrolysis of the furanone of step (3) with aqueous acid or base, preferably sodium hydroxide, to give compound **vb,** a thiol-protected (2R)-2-R¹-(4R)-4-hydroxy-5-mercaptopentanoic acid, where Z is triphenylmethyl, 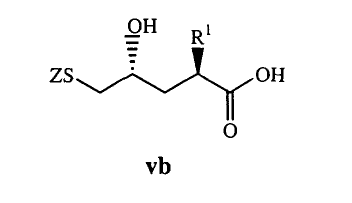
(5) protection of the hydroxy group of compound **vb** of step (4) with the t-butyldimethylsilyl protecting group to give compound **vib** where Y is t-butyldimethylsilyl, 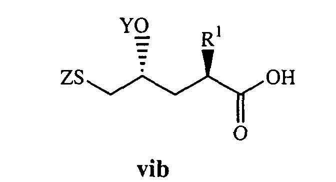
(6) amidation of carboxylic acid **vib** of step (5) with (2S)-t-butylglycine N-methylamide and the peptide coupling reagent diethylcyanophosphonate, and triethylamine, to provide compound **viib** where Y is t-butyldimethylsilyl and Z is triphenylmethyl, 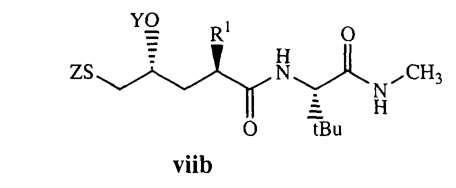
(7) removal of the t-butyldimethylsilyl protecting group Y from the hydroxyl group of compound **viib** of step (6) with tetrabutylammonium fluoride to provide alcohol **viiib,** where Z is triphenylmethyl, 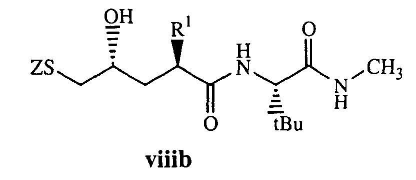
(8) removal of the triphenylmethyl thiol protecting group of compound **viiib** of step (7) with trifluoroacetic acid and triethylsilane to provide a compound of the above formula.

17. A process for the preparation of a compound of formula 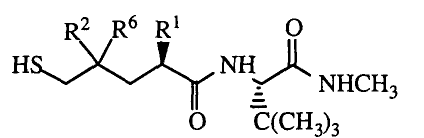 according to claim 1, where R¹ is as defined in Claim 1; and R² and R⁶ together form carbonyl which comprises:
(1) oxidation of compound of the formula 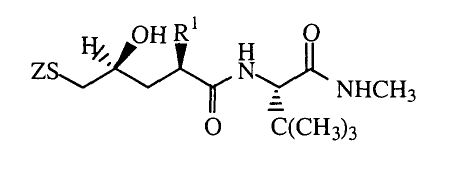 or 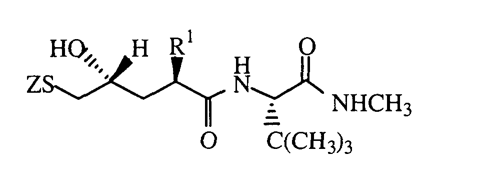 where R¹, R² and R⁶ are as previously defined, Z is a thiol protecting group,
and
(2) removal of the thiol protecting group to give a compound where R² and R⁶ together forms carbonyl.

18. The process according to claim 17 which compises
(1) oxidation of compound of the formula 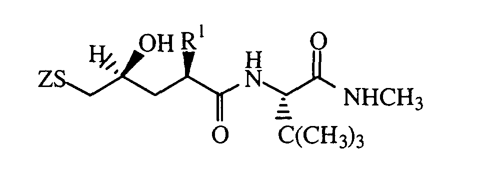 or 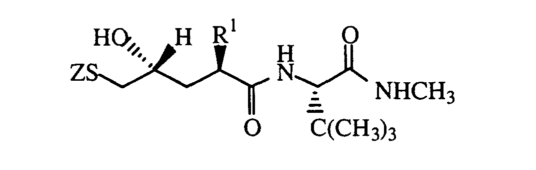 where R¹, R² and R⁶ are as previously defined, Y is hydrogen and Z triphenylmethyl,with dimethylsulfoxide/ pyridine/ trifluoroacetic acid/ 1-(3-dimethylaminopropyl)-3-ethylcarbodiimide or equivalent activated dimethylsulfoxide oxidation or other oxidative methods including Dess-Martin reagent, pyridinium dichromate, pyridinium chlorochromate/sodium acetate, tetrapropylammonium perruthenate/NMO
and
(2) removal of the triphenylmethyl thiol protecting group to give a compound where R² and R⁶ together forms carbonyl and Z is H.

19. A process for the preparation of an intermediate of the formula **ivb** 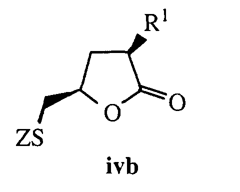 where R¹ is as defined in Claim 1 and Z is H or a thiol protecting group,
which comprises:
(1) reacting a compound of the formula **i** 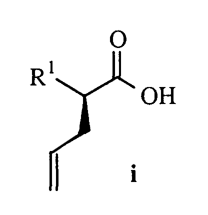 with iodine to obtain predominantly the (3*R*, 5*R*) dihydrofuranone of the formula **iib** 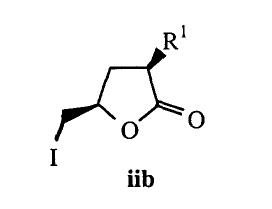
(2) reacting 3(*R*)-3-R¹-5(*R*)-iodomethyldihydrofuran-2-one of step (1) with a sulfur reagent, HSW, and a base where W is a thiol protecting group to obtain an intermediate of the formula **iiib,** 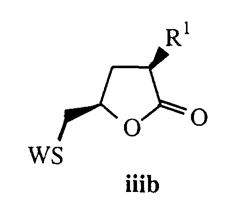
(3) deprotection of the sulfur of lactone **iiib** of step (2) under reducing conditions, acid hydrolysis or base hydrolysis, to provide compound **ivb',** 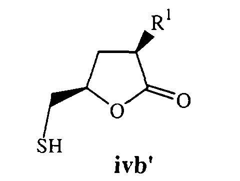 and
(4) protection of the free thiol of step (3) above to provide compound **ivb** where Z is H or a thiol protecting group.

20. The process according to claim 19 for the preparation of an intermediate of the formula **ivb** 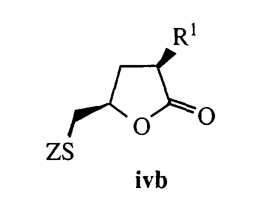 where R¹ is as defined above and Z is H or a thiol protecting group,
which comprises:
(1) reacting a compound of the formula **i** 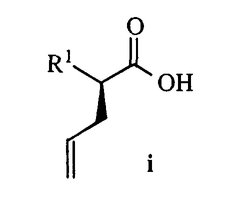 with iodine to obtain predominantly the (3*R*, 5*R*) dihydrofuranone of the formula **iib** 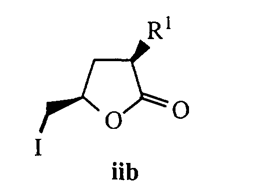
(2) reacting 3(*R*)-3-R¹-5(*R*)-iodomethyldihydrofuran-2-one of step (1) thiolacetic acid and sodium methoxide to obtain an intermediate of the formula **iiib** in which W is an acetyl group, 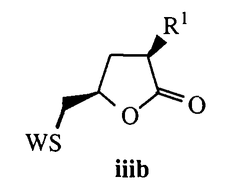
(3) deprotection of the sulfur of lactone **iiib** of step (2) where W is acetyl by reaction with sodium borohydride in methanol or ethanol, to provide compound **ivb',** 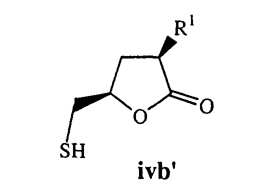 and
(4) protection of the free thiol of step (3) above to provide compound **ivb** where Z is triphenylmethyl.

21. A process for the preparation of an intermediate of the formula **iva** 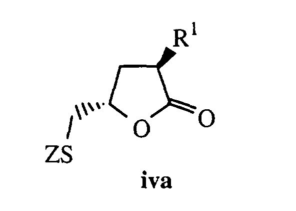 where R¹ is as defined in Claim 1 and Z is H or thiol protecting group which comprises:
(1) reacting a compound of the formula **i** 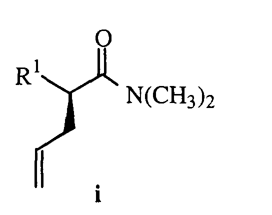 with iodine to obtain predominantly the (3*R*, 5*S*) dihydrofuranone of the formula **iia** 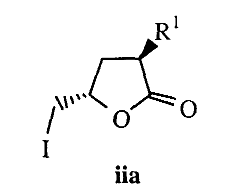
(2) reacting 3(*R*)-3-R¹-5(*S*)-iodomethyldihydrofuran-2-one of step (1) with a sulfur reagent, HSW, and a base where W is a thiol protecting group to obtain an intermediate of the formula **iiia**, 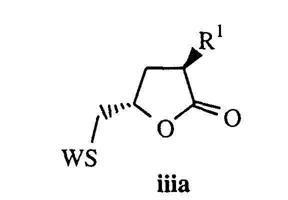
(3) deprotection of the sulfur of lactone **iiia** of step (2) under reducing conditions, acid hydrolysis or base hydrolysis, to provide compound **iva',** 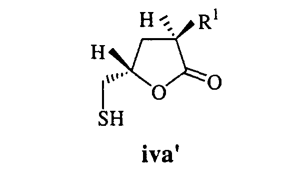 and
(4) protection of the free thiol of step (3) above to provide compound **iva** where Z is H or a thiol protecting group.

22. The process according to claim 21 for the preparation of an intermediate of the formula **iva** 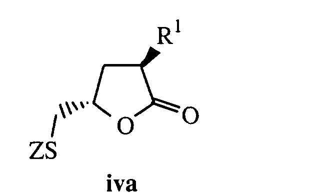 where R¹ is as defined in Claim 1 and Z is H,or a thiol protecing group, which comprises:
(1) reacting a compound of the formula **i** 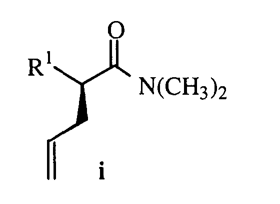 with iodine to obtain predominantly the (3*R*, 5*S*) dihydrofuranone of the formula **iia** 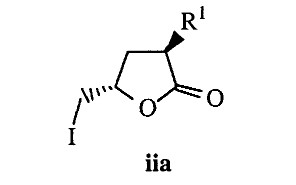
(2) reacting 3(*R*)-3-R¹-5(*S*)-iodone of step (1) thiolacetic acid and sodium methoxideto obtain an intermediate of the formula **iiia** in which W is an acetyl group, 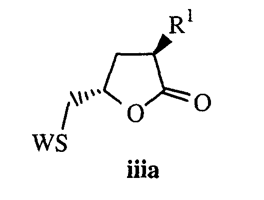
(3) deprotection of the sulfur of lactone **iiia** of step (2) where W is acetyl by reaction with sodium borohydride in methanol or ethanol, to provide compound **iva',** 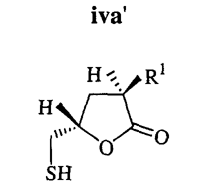 and
(4) protection of the free thiol of step (3) above to provide compound **iva** where Z is triphenylmethyl.

23. A compound according to Claim 1 which is (2*R*)-2-((2*S*)-2-Hydroxy-3-mercaptopropyl)nonanoic acid ((1*S*)-2,2-dimethyl-1-methylcarbamoylpropyl)amide or (2R,4*S*)-4-Hydroxy-2-isobutyl-5-mercaptopentanoic acid ((1*S*)-2,2-dimethyl-1-methylcarbamoylpropyl)amide.

24. A pharmaceutical composition comprising a compound as claimed in Claim 1 or Claim 23 and a pharmaceutically acceptable carrier.

## Patentansprüche

1. Diastereomerisch und enantiomerisch reine Verbindung gemäß der Formel: 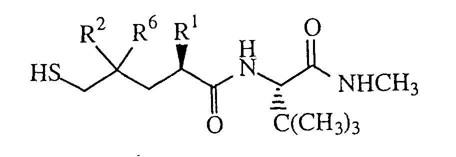
worin R¹ für C₁-C₁₂-Alkyl steht, grade oder verzweigt und gegebenenfalls substituiert durch Halogen, Hydroxy, C₁-C₆-Alkoxy, Amino, Carboxyl, C₁-C₆-Alkoxycarbonyl, Carboxamido, Nitril, Mono- oder Di-(C₁-C₆)alkylamino, Thio, C₁-C₆-Alkylthio, Aryl, -O-Aryl oder -OCH₂-Aryl, worin Aryl gegebenenfalls substituiert ist mit C₁-C₆-Alkyl, C₁-C₆-Alkoxy, Carboxy, Halogen, Cyano, Nitro, Carboxamido, Hydroxy oder C₁-C₆-Alkansulfonyl und Aryl eine 5- bis 10- gliedrige carbocyclische oder heterocyclische, mono- oder bicyclische aromatische Gruppe ist;
und R² für α-OH oder β-OH steht und R⁶ für H steht oder R² und R⁶ zusammen Carbonyl bilden.

2. Diastereomerisch und enantiomerisch reine Verbindung gemäß der Formel: 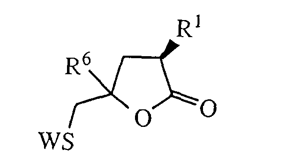
worin R¹ wie in Anspruch 1 definiert ist;
W eine Thiol-Schutzgruppe darstellt und
R⁶ für α-H oder β-H steht.

3. Verbindung gemäß Anspruch 2, worin W Acetyl oder Triphenylmethyl darstellt.

4. Diastereomerisch und enantiomerisch reine Verbindung gemäß der Formel: 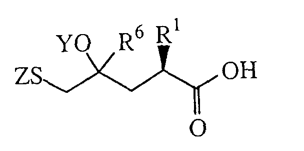
worin Y für H oder eine Hydroxyl-Schutzgruppe steht;
Z für H oder eine Thiol-Schutzgruppe steht;
R¹ wie in Anspruch 1 definiert ist und
R⁶ für α-H oder β-H steht.

5. Diastereomerisch und enantiomerisch reine Verbindung gemäß der Formel: 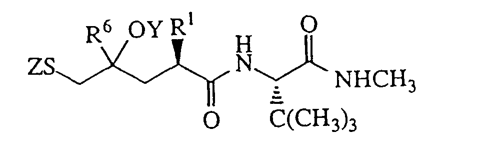
worin Y für H oder eine Hydroxyl-Schutzgruppe steht;
Z für H oder eine Thiol-Schutzgruppe steht;
R¹ wie in Anspruch 1 definiert ist und
R⁶ für α-H oder β-H steht.

6. Verbindung gemäß Anspruch 4 oder Anspruch 5, worin Y für tert-Butyldimethylsilyl steht und Z Trityl darstellt.

7. Verbindung gemäß Anspruch 4 oder Anspruch 5, worin Y für H steht und Z Trityl darstellt.

8. Diastereomerisch und enantiomerisch reine Verbindung gemäß der Formel: 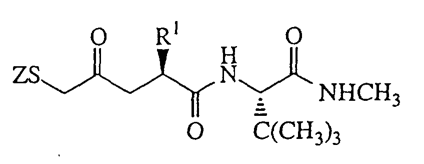
worin Z wie in Anspruch 5 definiert ist, unter der Voraussetzung, dass Z nicht für H steht; und
R¹ wie in Anspruch 1 definiert ist.

9. Verbindung gemäß Anspruch 8, worin Z für Trityl steht.

10. Verbindung gemäß einem der Ansprüche 1 bis 9, worin R¹ Heptyl oder Isobutyl darstellt.

11. Verfahren für die Herstellung einer diastereomerisch und enantiomerisch reinen Verbindung der Formel 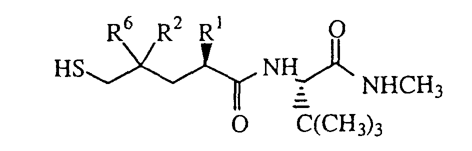 gemäß Anspruch 1, worin R¹ wie in Anspruch 1 definiert ist; R² für (S)-OH steht und R⁶ für H steht, welches umfasst: Entschützen einer Verbindung **viiia** 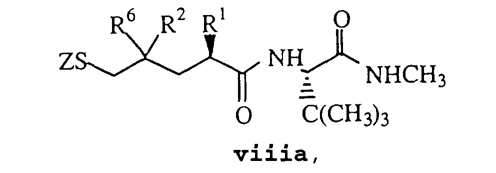 worin Z eine Thiol-Schutzgruppe darstellt.

12. Verfahren zur Herstellung einer diastereomerisch und enantiomerisch reinen Verbindung der Formel 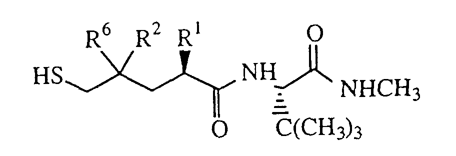 gemäß Anspruch 1, worin R¹ wie in Anspruch 1 definiert ist; R² für (*S*)-OH steht und R⁶ für H steht, welches umfasst:
(1) Umsetzen eines *(3R,5S*)-5-Iodomethyl-3-R¹-dihydrofuran-2-ons mit dem Anion von HSW, worin W eine Thiol-Schutzgruppe darstellt, um Lacton **iiia** vorzusehen 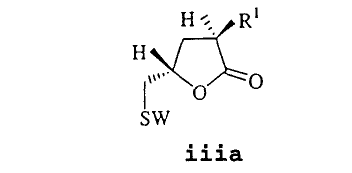
(2) Entschützen des Schwefels von Lacton **iiia** von Schritt (1) unter Reduktionsbedingungen, Säurehydrolyse oder Basehydrolyse, um Verbindung **iva'** vorzusehen, 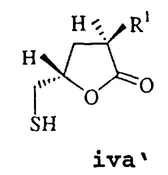
(3) Schützen des freien Thiols von Schritt (2) oben, um Verbindung **iva** vorzusehen, worin Z eine Thiol-Schutzgruppe darstellt, 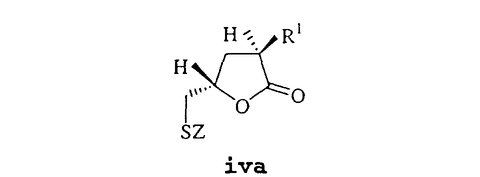
(4) Hydrolyse des Furanons von Schritt (3) mit wässeriger Säure oder Base, um Verbindung **va** 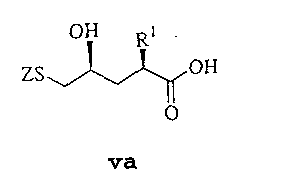 zu ergeben, eine Thiol-geschützte (2R)-2-R¹-(4R)-4-hydroxy-5-mercaptopentansäure, worin Z wie oben definiert ist,
(5) Schützen der Hydroxygruppe von Verbindung **va** von Schritt (4) mit einer Schutzgruppe Y, um die Verbindung **via** 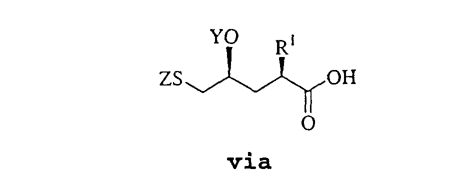 zu ergeben, worin Y eine Hydroxyl-Schutzgruppe darstellt,
(6) Amidieren von Carbonsäure **via** von Schritt (5) mit (2S)-t-Butylglycin-N-methylamid und einem Peptid-Kopplungsreagens, um Verbindung **viia** 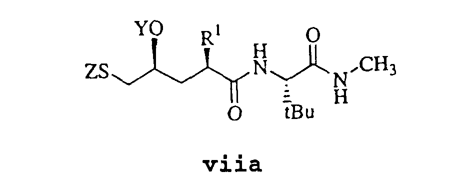 vorzusehen, worin Y und Z wie oben definiert sind,
(7) Entfernen der Schutzgruppe Y von der Hydroxylgruppe der Verbindung **viia** von Schritt (6), um Alkohol **viiia** 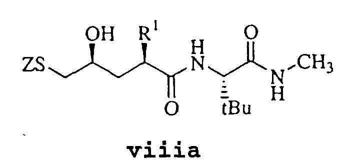 vorzusehen, worin Z wie oben definiert ist,
(8) Entfernen der Thiol-Schutzgruppe von Verbindung **viiia** von Schritt (7), um eine Verbindung der obigen Formel vorzusehen.

13. Verfahren gemäß Anspruch 12 für die Herstellung einer diastereomerisch und enantiomerisch reinen Verbindung der Formel 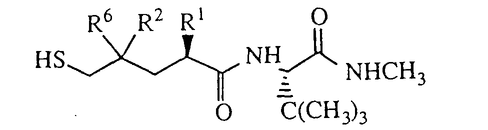 gemäß Anspruch 1, worin R¹ wie in Anspruch 1 definiert ist; R² für *(S)*-OH steht und R⁶ für H steht, welches umfasst:
(1) Umsetzen eines *(3R, 5S)*-5-Iodomethyl-3-R¹-dihydrofuran-2-ons mit dem Natrium-, Lithium- oder Kaliumsalz von Thiolessigsäure, um Lacton **iiia** vorzusehen, worin W Acetyl darstellt, 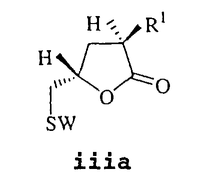
(2) Entschützen des Schwefels von Lacton **iiia** von Schritt (1), wobei die Acetylgruppe durch Behandlung mit Natriumborhydrid in Methanol oder Ethanol entfernt wird, um Verbindung **iva'** vorzusehen, 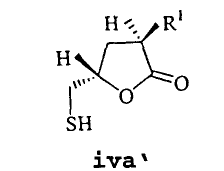
(3) Schützen des freien Thiols von Schritt (2) oben, um Verbindung **iva** vorzusehen, worin Z Triphenylmethyl darstellt, 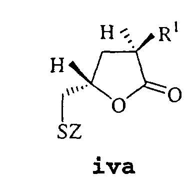
(4) Hydrolyse des Furanons von Schritt (3) mit wässeriger Säure oder Base, vorzugsweise Natriumhydroxid, um Verbindung **va** zu ergeben, eine Thiol-geschützte (2R)-2-R¹-(4R)-4-hydroxy-5-mercaptopentansäure, worin Z Triphenylmethyl darstellt, 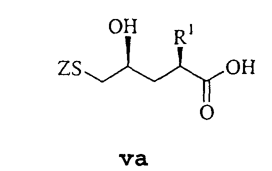
(5) Schützen der Hydroxygruppe von Verbindung **va** von Schritt (4) mit der t-Butyldimethylsilyl-Schutzgruppe, um die Verbindung **via** zu ergeben, worin Y t-Butyldimethylsilyl darstellt, 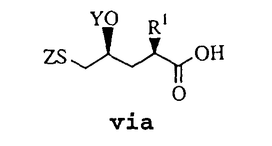
(6) Amidieren von Carbonsäure **via** von Schritt (5) mit (2S)-t-Butylglycin-N-methylamid und dem Peptid-Kopplungsreagens Diethylcyanophosphonat und Triethylamin, um Verbindung **viia** vorzusehen, worin Y t-Butyldimethylsilyl darstellt und Z Triphenylmethyl darstellt, 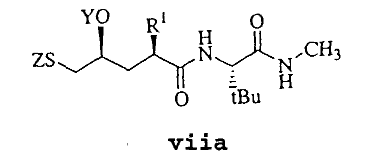
(7) Entfernen der t-Butyldimethylsilyl-Schutzgruppe Y von der Hydroxylgruppe der Verbindung **viia** von Schritt (6) mit Tetrabutylammoniumfluorid, um Alkohol **viiia** vorzusehen, worin Z Triphenylmethyl darstellt, 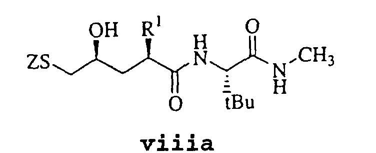
(8) Entfernen der Triphenylmethyl-Thiol-Schutzgruppe von Verbindung **viiia** von Schritt (7) mit Trifluoressigsäure und Triethylsilan, um eine Verbindung der obigen Formel vorzusehen.

14. Verfahren für die Herstellung einer Verbindung der Formel 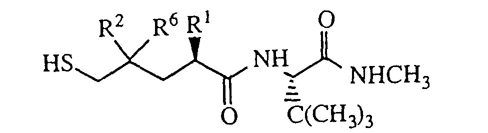 gemäß Anspruch 1, worin R¹ wie in Anspruch 1 definiert ist; R² für (*R*)-OH steht und R⁶ für H steht, welches umfasst: Entschützen einer Verbindung **viiib** 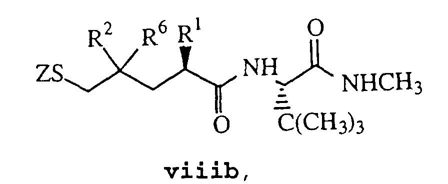 worin Z eine Thiol-Schutzgruppe darstellt.

15. Verfahren für die Herstellung einer Verbindung der Formel 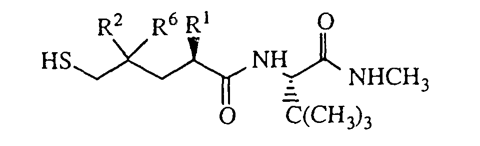 gemäß Anspruch 1, worin R¹ wie in Anspruch 1 definiert ist; R² für (R)-OH steht und R⁶ für H steht, welches umfasst:
(1) Umsetzen eines *(3R, 5R*)-5-Iodomethyl-3-R¹-dihydrofuran-2-ons mit dem Anion von HSW, worin W eine Thiol-Schutzgruppe darstellt, um Lacton **iiib** vorzusehen, 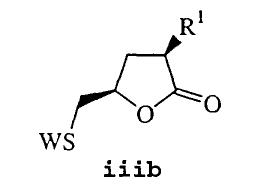
(2) Entschützen des Schwefels von Lacton **iiib** von Schritt (1) unter Reduktionsbedingungen, Säurehydrolyse oder Basehydrolyse, um Verbindung **ivb'** vorzusehen, 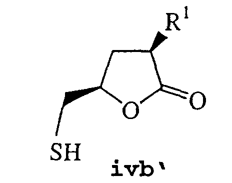
(3) Schützen des freien Thiols von Schritt (2) oben, um Verbindung **ivb** vorzusehen, worin Z eine Thiol-Schutzgruppe darstellt, 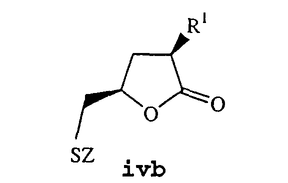
(4) Hydrolyse des Furanons von Schritt (3) mit wässeriger Säure oder Base, um Verbindung **va,** eine Thiol-geschützte (2R)-2-R¹-(4R)-4-Hydroxy-5-mercaptopentansäure zu ergeben, worin Z wie oben definiert ist, 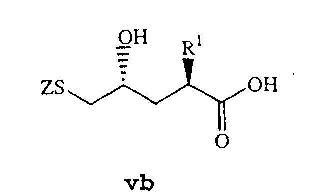
(5) Schützen der Hydroxygruppe von Verbindung **vb** von Schritt (4) mit einer Schutzgruppe Y, um Verbindung **vib** zu ergeben, worin Y eine Hydroxyl-Schutzgruppe darstellt 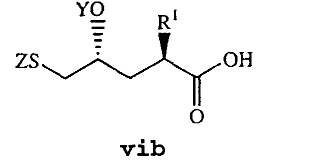
(6) Amidieren von Carbonsäure **vib** von Schritt (5) mit (2S)-t-Butylglycin-N-methylamid und einem Peptid-Kopplungsreagens, um Verbindung **viib** vorzusehen, worin Y und Z wie oben definiert sind, 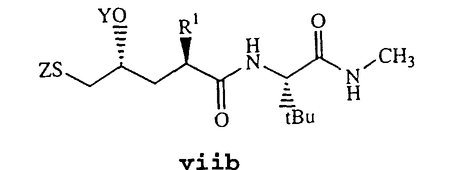
(7) Entfernen der Schutzgruppe Y von der Hydroxylgruppe der Verbindung **viib** von Schritt (6), um Alkohol **viiib** vorzusehen, worin Z wie oben definiert ist, 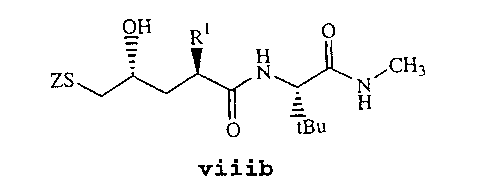
(8) Entfernen der Thiol-Schutzgruppe von Verbindung **viiib** von Schritt (7), um eine Verbindung der obigen Formel vorzusehen.

16. Verfahren gemäß Anspruch 15 für die Herstellung einer diastereomerisch und enantiomerisch reinen Verbindung der Formel 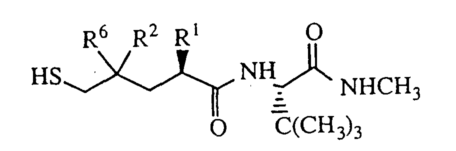 gemäß Anspruch 1, worin R¹ wie in Anspruch 1 definiert ist; R² für (*R*)-OH steht und R⁶ für H steht, welches umfasst:
(1) Umsetzen eines *(3R, 5R)*-5-Iodomethyl-3-R¹-dihydrofuran-2-ons mit dem Natrium-, Lithium- oder Kaliumsalz von Thiolessigsäure, um Lacton **iiib** vorzusehen, worin W Acetyl darstellt, 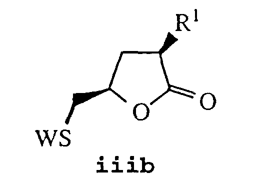
(2) Entschützen des Schwefels von Lacton **iiib** von Schritt (1), wobei die Acetylgruppe durch Behandlung mit Natriumborhydrid in Methanol oder Ethanol entfernt wird, um Verbindung **ivb'** vorzusehen, 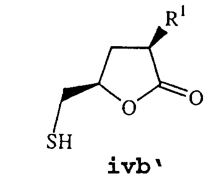
(3) Schützen des freien Thiols von Schritt (2) oben, um die Verbindung **ivb** vorzusehen, worin Z Triphenylmethyl darstellt, 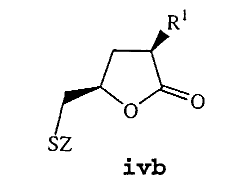
(4) Hydrolyse des Furanons von Schritt (3) mit wässeriger Säure oder Base, vorzugsweise Natriumhydroxid, um Verbindung **vb** zu ergeben, eine Thiol-geschützte (2R)-2-R¹-(4R)-4-Hydroxy-5-mercaptopentansäure, worin Z Triphenylmethyl darstellt, 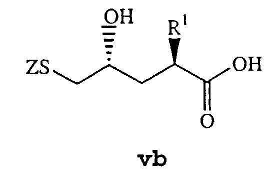
(5) Schützen der Hydroxygruppe von Verbindung **vb** von Schritt (4) mit der t-Butyldimethylsilyl-Schutzgruppe, um die Verbindung **vib** zu ergeben, worin Y t-Butyldimethylsilyl darstellt, 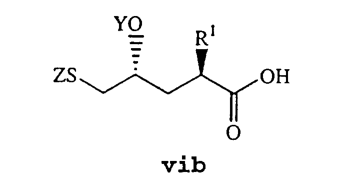
(6) Amidieren von Carbonsäure **vib** von Schritt (5) mit (2S)-t-Butylglycin-N-methylamid und dem Peptid-Kopplungsreagens Diethylcyanophosphonat und Triethylamin, um Verbindung **viib** vorzusehen, worin Y t-Butyldimethylsilyl darstellt und Z Triphenylmethyl darstellt, 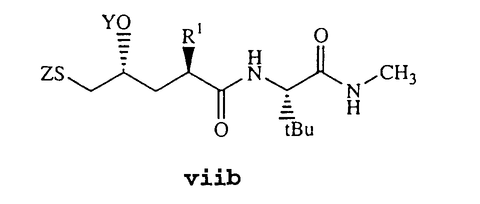
(7) Entfernen der t-Butyldimethylsilyl-Schutzgruppe Y von der Hydroxylgruppe der Verbindung **viib** von Schritt (6) mit Tetrabutylammoniumfluorid, um Alkohol **viiib** vorzusehen, worin Z Triphenylmethyl darstellt, 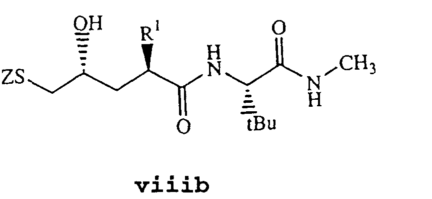
(8) Entfernen der Triphenylmethyl-Thiol-Schutzgruppe von Verbindung **viiib** von Schritt (7) mit Trifluoressigsäure und Triethylsilan, um eine Verbindung der obigen Formel vorzusehen.

17. Verfahren für die Herstellung einer Verbindung der Formel 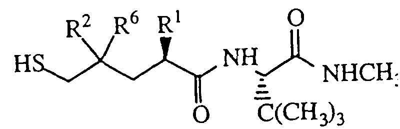 gemäß Anspruch 1, worin R¹ wie in Anspruch 1 definiert ist; und R² und R⁶ zusammen Carbonyl bilden, welches umfasst:
(1) Oxidation von Verbindung der Formel 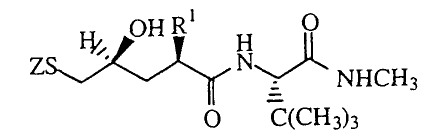 oder 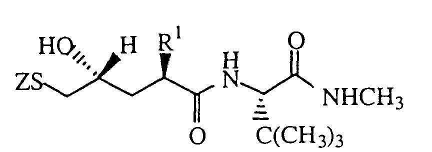 worin R¹, R² und R⁶ wie vorhergehend definiert sind, Z eine Thiol-Schutzgruppe darstellt, und
(2) Entfernen der Thiol-Schutzgruppe, um eine Verbindung zu ergeben, worin R² und R⁶ zusammen Carbonyl bilden.

18. Verfahren gemäß Anspruch 17, welches umfasst:
(1) Oxidation von Verbindung der Formel 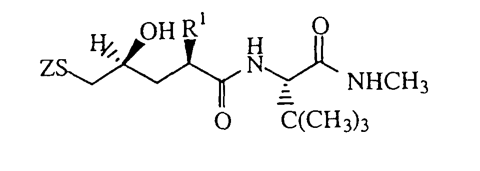 oder 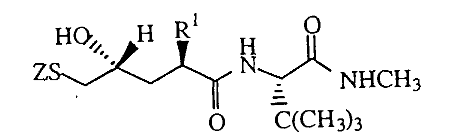 worin R¹, R² und R⁶ wie vorhergehend definiert sind, Y Wasserstoff darstellt und Z Triphenylmethyl darstellt, mit Dimethylsulfoxid/Pyridin/Trifluoressigsäure/1-(3-Dimethylaminopropyl)-3-ethylcarbodiimid oder äquivalent aktivierter Dimethylsulfoxid-Oxidation oder anderen oxidativen Verfahren einschließlich Dess-Martin-Reagens, Pyridiniumdichromat, Pyridiniumchlorchromat/Natriumacetat, Tetrapropylammoniumperruthenat/NMO, und
(2) Entfernen der Triphenylmethyl-Thiol-Schutzgruppe, um eine Verbindung zu ergeben, worin R² und R⁶ zusammen Carbonyl bilden und Z für H steht.

19. Verfahren für die Herstellung einer Zwischenverbindung der Formel **ivb** 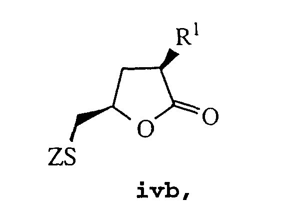 worin R¹ wie in Anspruch 1 definiert ist und Z für H oder eine Thiol-Schutzgruppe steht, welches umfasst:
(1) Umsetzen einer Verbindung der Formel **i** 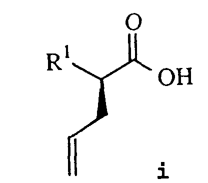 mit Iod, um überwiegend das *(3R,5R)*-Dihydrofuranon der Formel **iib** 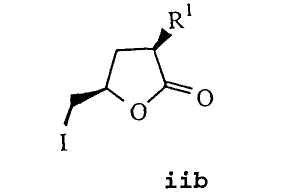 zu erhalten,
(2) Umsetzen von 3*(R)*-3-R¹-5*(R)*-Iodmethyldihydrofuran-2-on von Schritt (1) mit einem Schwefelreagens, HSW, und einer Base, worin W eine Thiol-Schutzgruppe darstellt, um eine Zwischenverbindung der Formel **iiib** 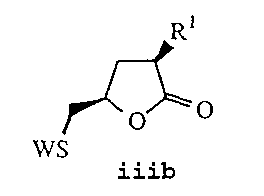 zu erhalten,
(3) Entschützen des Schwefels von Lacton **iiib** von Schritt (2) unter Reduktionsbedingungen, Säurehydrolyse oder Basehydrolyse, um Verbindung **ivb'** 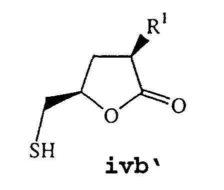 vorzusehen, und
(4) Schützen des freien Thiols von Schritt (3) oben, um Verbindung **ivb** vorzusehen, worin Z für H oder eine Thiol-Schutzgruppe steht.

20. Verfahren gemäß Anspruch 19 für die Herstellung einer Zwischenverbindung der Formel **ivb** 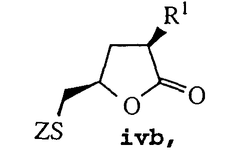 worin R¹ wie oben definiert ist und Z für H oder eine Thiol-Schutzgruppe steht, welches umfasst:
(1) Umsetzen einer Verbindung der Formel **i** 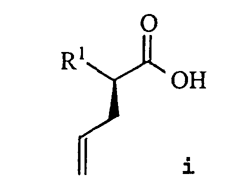 mit Iod, um überwiegend das *(3R,5R)*-Dihydrofuranon der Formel **iib** 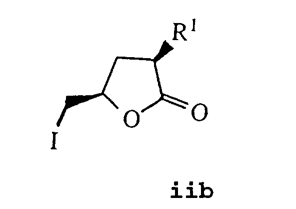 zu erhalten,
(2) Umsetzen von 3(*R*)-3-R¹-5(*R*)-Iodmethyldihydrofuran-2-on von Schritt (1), Thiolessigsäure und Natriummethoxid, um eine Zwischenverbindung der Formel **iiib** zu erhalten, worin W eine Acetylgruppe darstellt, 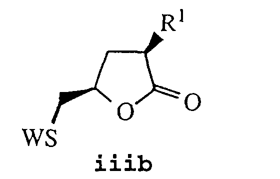
(3) Entschützen des Schwefels von Lacton **iiib** von Schritt (2), worin W Acetyl darstellt, durch Reduktion mit Natriumborhydrid in Methanol oder Ethanol, um Verbindung **ivb'** 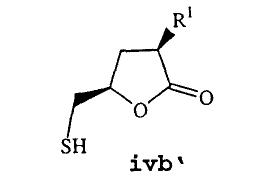 vorzusehen, und
(4) Schützen des freien Thiols von Schritt (3) oben, um Verbindung **ivb** vorzusehen, worin Z für Triphenylmethyl steht.

21. Verfahren für die Herstellung einer Zwischenverbindung der Formel **iva** 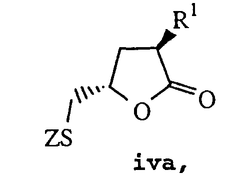 worin R¹ wie in Anspruch 1 definiert ist und Z für H oder Thiol-Schutzgruppe steht, welches umfasst:
(1) Umsetzen einer Verbindung der Formel **i** 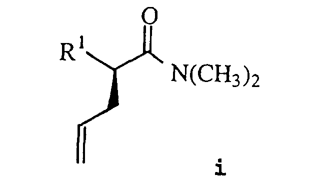 mit Iod, um überwiegend das *(3R,5S)*-Dihydrofuranon der Formel **iia** 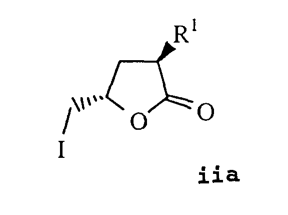 zu erhalten,
(2) Umsetzen von 3*(R)*-3-R¹-5*(S)*-Iodmethyldihydrofuran-2-on von Schritt (1) mit einem Schwefelreagens, HSW, und einer Base, worin W eine Thiol-Schutzgruppe darstellt, um eine Zwischenverbindung der Formel **iiia** 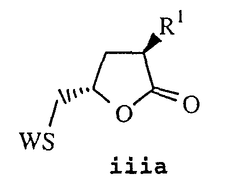 zu erhalten,
(3) Entschützen des Schwefels von Lacton **iiia** von Schritt (2) unter Reduktionsbedingungen, Säurehydrolyse oder Basehydrolyse, um Verbindung **iva'** 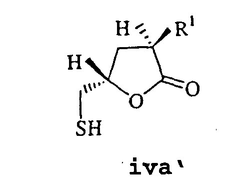 vorzusehen, und
(4) Schützen des freien Thiols von Schritt (3) oben, um Verbindung **iva** vorzusehen, worin Z für H oder eine Thiol-Schutzgruppe steht.

22. Verfahren gemäß Anspruch 21 für die Herstellung einer Zwischenverbindung der Formel **iva** 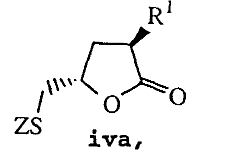 worin R¹ wie in Anspruch 1 definiert ist und Z für H oder eine Thiol-Schutzgruppe steht, welches umfasst:
(1) Umsetzen einer Verbindung der Formel **i** 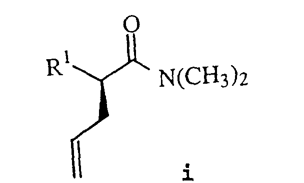 mit Iod, um überwiegend das *(3R, 5S)*-Dihydrofuranon der Formel **iia** 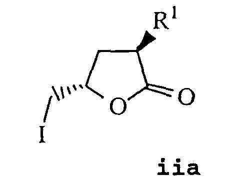 zu erhalten,
(2) Umsetzen von 3*(R)*-3-R¹-5*(S)*-Iodon von Schritt (1), Thiolessigsäure und Natriummethoxid, um eine Zwischenverbindung der Formel **iiia** zu erhalten, worin W eine Acetylgruppe darstellt, 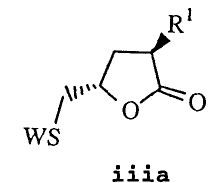
(3) Entschützen des Schwefels von Lacton **iiia** von Schritt (2), worin W Acetyl darstellt, durch Umsetzung mit Natriumborhydrid in Methanol oder Ethanol, um Verbindung **iva'** 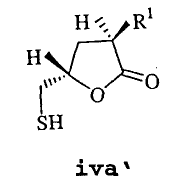 vorzusehen, und
(4) Schützen des freien Thiols von Schritt (3) oben, um Verbindung **iva** vorzusehen, worin Z für Triphenylmethyl steht.

23. Verbindung gemäß Anspruch 1, welche *(2R*)-2-(*(2S)*-2-Hydroxy-3-mercaptopropyl)nonansäure ((*1S)-2,2-dimethyl-1-methylcarba*moylpropyl)amid oder *(2R, 4S)*-4-Hydroxy-2-isobutyl-5-mercaptopentansäure (*(1S)*-2,2-dimethyl-1-methylcarbamoylpropyl)amid ist.

24. Pharmazeutische Zusammenfassung, umfassend eine Verbindung wie in Anspruch 1 oder Anspruch 23 beansprucht, und einen pharmazeutisch annehmbaren Träger.

## Revendications

1. Composé diastéréoisomériquement et énantiomériquement pur selon la formule : 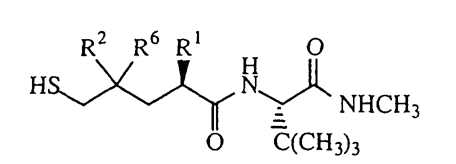
dans lequel R¹ est un alkyle en C₁ à C₁₂, linéaire ou ramifié et facultativement substitué par un halogène, un hydroxy, un alkoxy en C₁ à C₆, un amino, un carboxyle, un alkoxycarbonyle en C₁ à C₆, un carboxamido, un nitrile, un mono- ou di(alkyle en C₁ à C₆)amino, un thio, un alkylthio en C₁ à C₆, un aryle, un -Oaryle ou un -OCH₂aryle, où l'aryle est facultativement substitué par un alkyle en C₁ à C₆, un alkoxy en C₁ à C₆, un carboxy, un halogène, un cyano, un nitro, un carboxamido, un hydroxy ou un alcane(en C₁ à C₆)sulfonyle, et l'aryle est un groupe aromatique carbocyclique ou hétérocyclique, mono ou bicyclique comptant de 5 à 10 chaînons;
et R² est un α-OH ou un β-OH, et R⁶ est H ou R² et R⁶ forment ensemble un carbonyle.

2. Composé diastéréoisomériquement et énantiomériquement pur selon la formule : 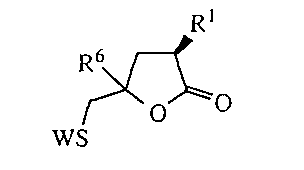 dans lequel R¹ est comme défini dans la revendication 1; W est un groupe de protection du thiol et R⁶ est un α-H ou un β-H.

3. Composé selon la revendication 2 dans lequel W est un acétyle ou un triphénylméthyle.

4. Composé diastéréoisomériquement et énantiomériquement pur selon la formule : 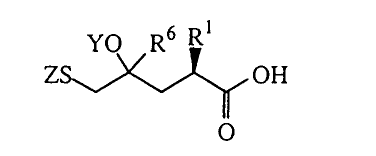
dans lequel Y est H ou un groupe de protection de l'hydroxyle;
Z est H ou un groupe de protection du thiol;
R¹ est tel que défini dans la revendication 1, et
R⁶ est un α-H ou un β-H.

5. Composé diastéréoisomériquement et énantiomériquement pur selon la formule : 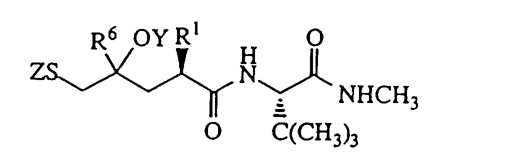
dans lequel Y est H ou un groupe de protection de l'hydroxyle;
Z est H ou un groupe de protection du thiol;
R¹ est tel que défini dans la revendication 1, et
R⁶ est un α-H ou un β-H.

6. Composé selon la revendication 4 ou la revendication 5, dans lequel Y est le tert-butyldiméthylsilyle et Z est le trityle.

7. Composé selon la revendication 4 ou la revendication 5, dans lequel Y est H et Z est le trityle.

8. Composé diastéréoisomériquement et énantiomériquement pur selon la formule : 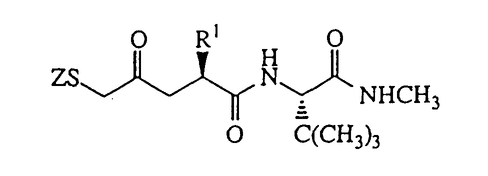
dans lequel Z est tel que défini dans la revendication 5 avec la condition que Z n'est pas H;
et R¹ est tel que défini dans la revendication 1.

9. Composé selon la revendication 8, dans lequel Z est le trityle.

10. Composé selon l'une quelconque des revendications 1 à 9, dans lequel R¹ est l'heptyle ou l'isobutyle.

11. Procédé pour la préparation d'un composé diastéréoisomériquement et énantiomériquement pur de formule 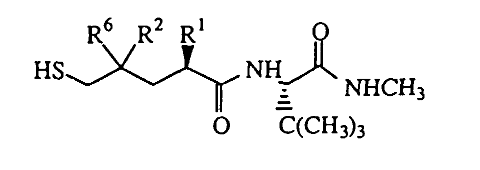 selon la revendication 1,
dans lequel R¹ est tel que défini dans la revendication 1;
R² est un *(S)*-OH et R⁶ est H,
qui comprend l'étape consistant à déprotéger un composé **viiia** 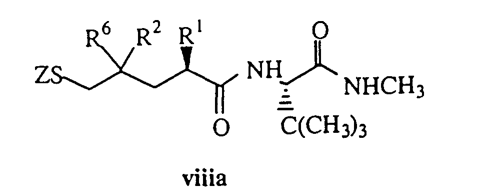 dans lequel Z est un groupe de protection du thiol.

12. Procédé pour la préparation d'un composé diastéréoisomériquement et énantiomériquement pur de formule 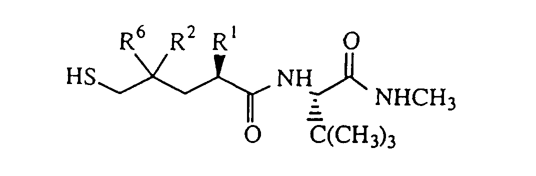 selon la revendication 1, dans lequel
R¹ est tel que défini dans la revendication 1; R² est *(S)*-OH et R⁶ est H,
qui comprend les étapes consistant à:
(1) faire réagir une *(3R, 5S)*-5-iodométhyl-3-R¹-dihydrofuran-2-one avec l'anion de HSW, où W est un groupe de protection du thiol, pour fournir une lactone **iiia,** 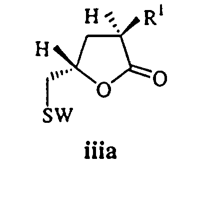
(2) déprotéger le soufre de la lactone **iiia** de l'étape
(1) dans des conditions de réduction, d'hydrolyse acide ou d'hydrolyse basique pour fournir le composé **iva',** 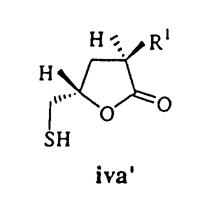
(3) protéger le thiol libre de l'étape (2) ci-dessus pour fournir le composé **iva** dans lequel Z est un groupe de protection du thiol, 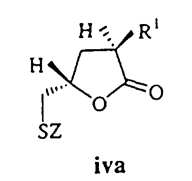
(4) hydrolyser la furanone de l'étape (3) par un acide ou une base en solution aqueuse pour donner le composé **va**, 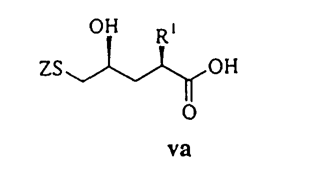 un acide (2R)-2-R¹-(4R)-4-hydroxy-5-mercaptopentanoïque à thiol protégé, dans lequel Z est tel que défini plus haut,
(5) protéger le groupe hydroxy du composé **va** de l'étape (4) par un groupe de protection Y pour donner le composé **via**, 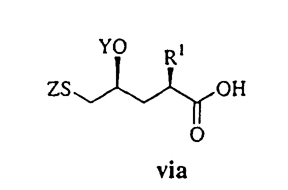 dans lequel Y est un groupe de protection de l'hydroxyle,
(6) amider l'acide carboxylique **via** de l'étape (5) par le N-méthylamide de (2S)-t-butylglycine et un réactif de couplage au peptide pour fournir le composé **viia,** 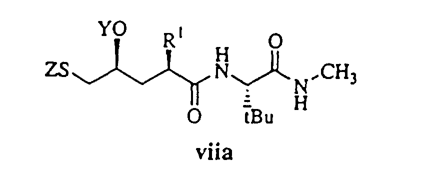 dans lequel Y et Z sont tels que définis plus haut,
(7) enlever le groupe de protection Y du groupe hydroxyle du composé **viia** de l'étape (6) pour fournir un alcool **viiia,** 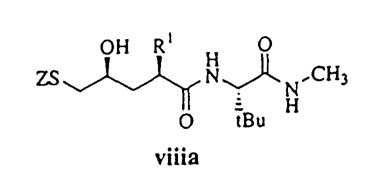 dans lequel Z est tel que défini plus haut,
(8) enlever le groupe de protection du thiol de composé **viiia** de l'étape (7) pour fournir un composé du formule ci-dessus.

13. Procédé selon la revendication 12 pour la préparation d'un composé diastéréoisomériquement et énantiomériquement pur de formule 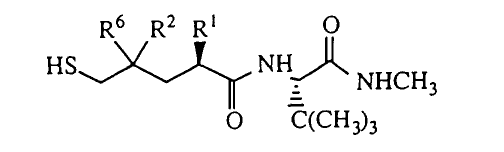 selon la revendication 1,
dans lequel R¹ est tel que défini dans la revendication 1; R² est un *(S)*-OH et R⁶ est H,
qui comprend les étapes consistant à:
(1) faire réagir une *(3R, 5S)*-5-iodométhyl-3-R¹-dihydrofuran-2-one avec le sel de sodium, de lithium ou de potassium de l'acide thioacétique pour fournir la lactone **iiia,** W étant l'acétyle, 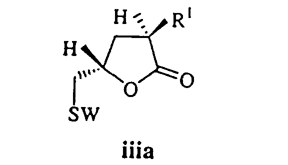
(2) déprotéger le soufre de la lactone **iiia** de l'étape (1), le groupe acétyle étant enlevé par traitement au borohydrure de sodium dans le méthanol ou l'éthanol, pour fournir le composé **iva',** 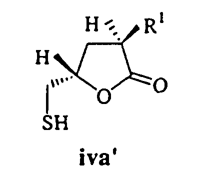
(3) protéger le thiol libre de l'étape (2) ci-dessus pour fournir le composé **iva** dans lequel Z est le triphénylméthyle, 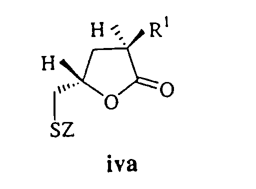
(4) hydrolyser la furanone de l'étape (3) par un acide ou une base en solution aqueuse, de préférence par l'hydroxyde de sodium pour donner le composé **va**, un acide (2R)-2-R¹-(4R)-4-hydroxy-5-mercaptopentanoique à thiol protégé dans lequel Z est le triphénylméthyle, 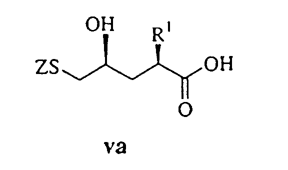
(5) protéger le groupe hydroxy du composé **va** de l'étape (4) par le groupe de protection t-butyldiméthylsilyle pour donner le composé **via** dans lequel Y est le t-butyldiméthylsilyle, 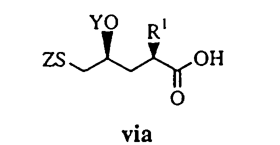
(6) amider l'acide carboxylique via de l'étape (5) par le N-méthylamide de (2S)-t-butylglycine, le réactif de couplage peptidique cyanophosphonate de diéthyle et la triéthylamine, pour fournir le composé **viia** dans lequel Y est le t-butyldiméthylsilyle et Z est le triphénylméthyle, 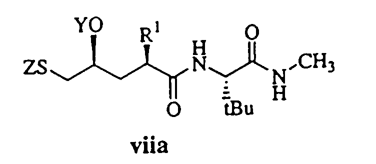
(7) enlever le groupe de protection Y t-butyldiméthylsilyle du groupe hydroxyle du composé **viia** de l'étape (6) par le fluorure de tétrabutylammonium pour fournir l'alcool **viiia** dans lequel Z est le triphénylméthyle, 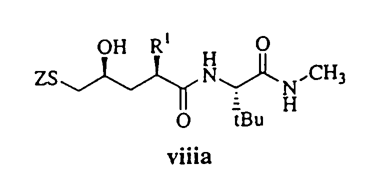
(8) enlever le groupe de protection triphénylméthyle du thiol du composé viiia de l'étape (7) par l'acide trifluoroacétique et le triéthylsilane pour fournir un composé de formule ci-dessus.

14. Procédé de préparation d'un composé de formule 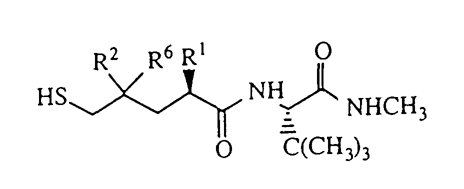 selon la revendication 1,
dans lequel R¹ est tel que défini dans la revendication 1; R² est (*R*)-OH et R⁶ est H,
qui comprend la déprotection d'un composé **viiib** 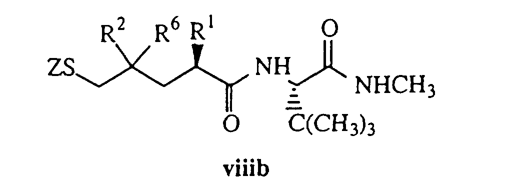 dans lequel Z est un groupe de protection du thiol.

15. Procédé de préparation d'un composé de formule 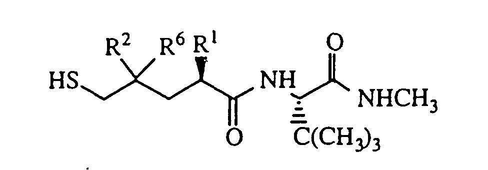 selon la revendication 1,
dans lequel R¹ est tel que défini dans la revendication 1; R² est *(R)*-OH et R⁶ est H,
qui comprend les étapes consistant à:
(1) faire réagir une *(3R, 5R)*-5-iodométhyl-3-R¹-dihydrofuran-2-one avec l'anion de HSW, W étant un groupe de protection du thiol, pour fournir une lactone **iiib**, 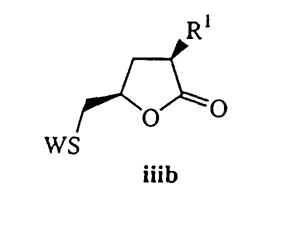
(2) déprotéger le soufre de la lactone **iiib** de l'étape (1) dans des conditions réductrices, par hydrolyse acide ou hydrolyse basique pour fournir le composé **ivb'**, 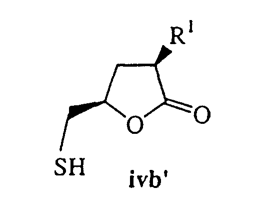
(3) protéger le thiol libre de l'étape (2) ci-dessus pour fournir le composé **ivb** dans lequel Z est un groupe de protection du thiol, 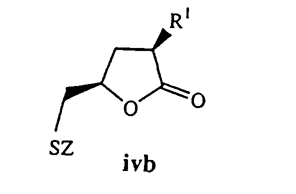
(4) hydrolyser la furanone de l'étape (3) par un acide ou une base en solution aqueuse pour donner le composé **vb,** un acide (2R)-2-R¹-(4R)-4-hydroxy-5-mercaptopentanoïque à thiol protégé, dans lequel Z est tel que défini plus haut, 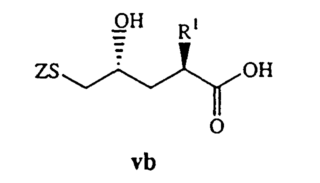
(5) protéger le groupe hydroxy du composé **vb** de l'étape (4) par un groupe de protection Y pour donner le composé **vib** où Y est un groupe de protection de l'hydroxyle, 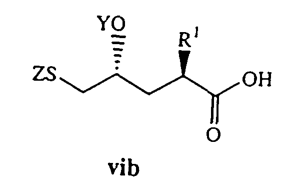
(6) amider l'acide carboxylique vib de l'étape (5) par le N-méthylamide de (2S)-t-butylglycine et un agent de couplage peptidique pour fournir le composé **viib** dans lequel Y et Z sont tels que définis plus haut, 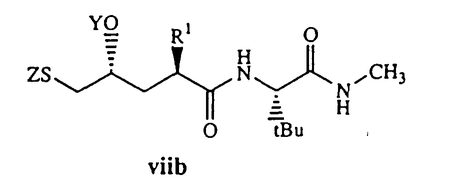
(7) enlever le groupe de protection Y du groupe hydroxyle du composé **viib** de l'étape (6) pour fournir l'alcool **viiib,** dans lequel Z est tel que défini plus haut, 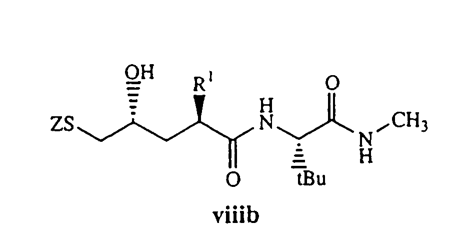
(8) enlever le groupe de protection du thiol du composé **viiib** de l'étape (7) pour fournir un composé de formule ci-dessus.

16. Procédé selon la revendication 15, pour la préparation d'un composé diastéréoisomériquement et énantiomériquement pur de formule : 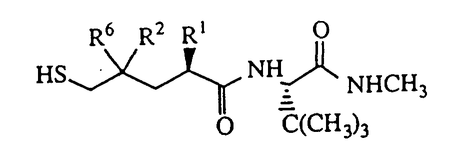 selon la revendication 1,
dans lequel R¹ est tel que défini dans la revendication 1; R² est *(R)*-OH et R⁶ est H,
qui comprend les étapes consistant à:
(1) faire réagir une *(3R,5R)*-5-iodométhyl-3-R¹-dihydrofuran-2-one avec le sel de sodium, de lithium ou de potassium de l'acide thioacétique pour fournir une lactone **iiib**, W étant l'acétyle, 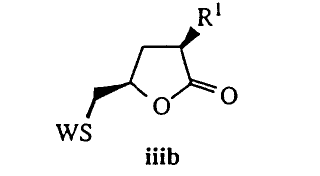
(2) déprotéger le soufre de la lactone **iiib** de l'étape (1), le groupe acétyle étant enlevé par traitement au borohydrure de sodium dans le méthanol ou l'éthanol, pour fournir le composé **ivb',** 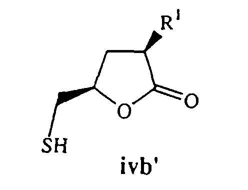
(3) protéger le thiol libre de l'étape (2) ci-dessus pour fournir le composé **ivb** dans lequel Z est le triphénylméthyle, 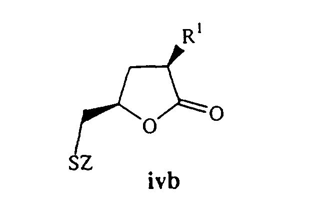
(4) hydrolyser la furanone de l'étape (3) par un acide ou une base en solution aqueuse, de préférence l'hydroxyde de sodium, pour donner le composé **vb**, un acide (2R)-2-R¹-(4R)-4-hydroxy-5-mercaptopentanoïque à thiol protégé dans lequel Z est le triphénylméthyle, 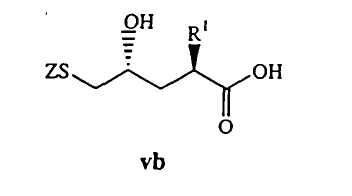
(5) protéger le groupe hydroxy du composé **vb** de l'étape (4) par le groupe de protection t-butyldiméthylsilyle pour donner le composé **vib** dans lequel Y est le t-butyldiméthylsilyle, 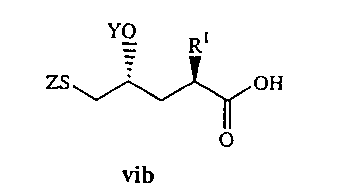
(6) amider l'acide carboxylique **vib** de l'étape (5) par le N-méthylamide de (2S)-t-butylglycine, le réactif de couplage peptidique cyanophosphonate de diéthyle et la triéthylamine, pour fournir le composé **viib** dans lequel Y est le t-butyldiméthylsilyle et Z est le triphénylméthyle, 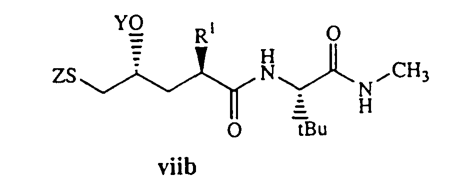
(7) enlever le groupe Y de protection t-butyldiméthylsilyle du groupe hydroxyle du composé **viib** de l'étape (6) par le fluorure de tétrabutylammonium pour fournir l'alcool **viiib** dans lequel Z est le triphénylméthyle, 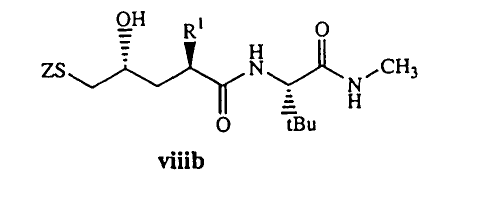
(8) enlever le groupe triphénylméthyle de protection du thiol du composé **viiib** de l'étape (7) par l'acide trifluoroacétique et le triéthylsilane pour fournir un composé de formule ci-dessus.

17. Procédé pour la préparation d'un composé de formule 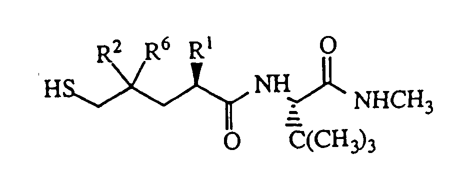 selon la revendication 1,
dans lequel R¹ est tel que défini dans la revendication 1; et R² et R⁶ forment ensemble un carbonyle,
qui comprend les étapes consistant à:
(1) oxyder le composé de formule 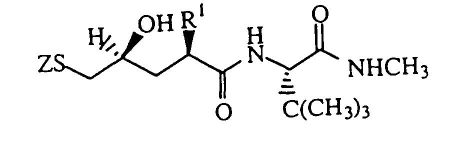 ou 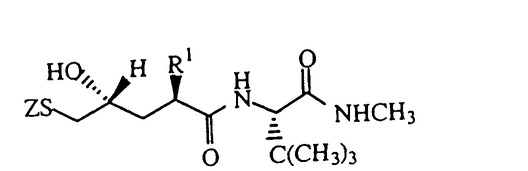 dans lesquels R¹, R² et R⁶ sont comme définis précédemment, Z étant un groupe de protection du thiol, et
(2) enlever le groupe de protection du thiol pour donner un composé dans lequel R² et R⁶ forment ensemble un carbonyle.

18. Procédé selon la revendication 17, qui comprend les étapes consistant à:
(1) oxyder le composé de formule : 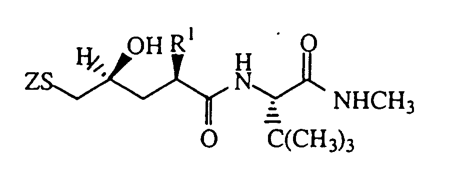 ou 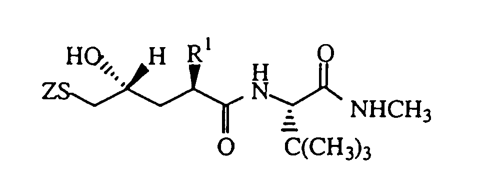 dans lesquels R¹, R² and R⁶ sont tels que définis précédemment, Y est l'hydrogène et Z le triphénylméthyle, avec une oxydation du sulfoxyde de diméthyle activée par sulfoxyde de diméthyle/pyridine/acide trifluoroacétique/1-(3-diméthylaminopropyl)-3-éthylcarbodiimide ou équivalent ou d'autres méthodes oxydantes comprenant le réactif de Dess-Martin, le dichromate de pyridinium, le chlorochromate de pyridinium/acétate de sodium, le perruthénate de tétrapropylammonium/NMO, et
(2) enlever le groupe triphénylméthyle de protection du thiol pour donner un composé dans lequel R² et R⁶ forment ensemble un carbonyle et Z est H.

19. Procédé pour la préparation d'un intermédiaire de formule **ivb :** 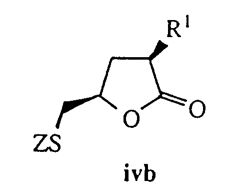 dans lequel R¹ est tel que défini dans la revendication 1, et Z est H ou un groupe de protection du thiol,
qui comprend les étapes consistant à:
(1) faire réagir un composé de formule **i** 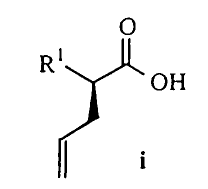 avec de l'iode pour obtenir de manière prédominante le (3*R*, *5R)*dihydrofuranone de formule **iib :** 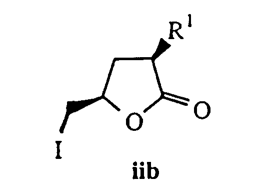
(2) faire réagir la 3*(R)*-3-R¹-5*(R)*-iodométhyldihydrofuran-2-one de l'étape (1) avec un réactif soufre, HSW et une base, W étant un groupe de protection du thiol, pour obtenir un intermédiaire de formule **iiib**, 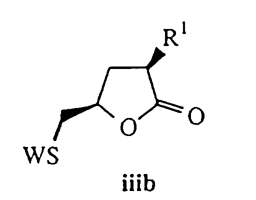
(3) déprotéger le soufre de la lactone **iiib** de l'étape (2) en conditions réductrices, par hydrolyse acide ou hydrolyse basique, pour fournir le composé **ivb',** 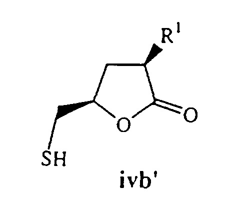 et
(4) protéger le thiol libre de l'étape (3) ci-dessus pour fournir un composé **ivb** dans lequel Z est H ou un groupe de protection du thiol.

20. Procédé selon la revendication 19 pour la préparation d'un intermédiaire de formule **ivb** 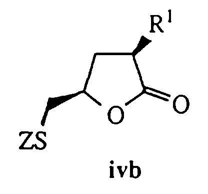 dans lequel R¹ est tel que défini plus haut et Z est H ou un groupe de protection du thiol,
qui comprend les étapes consistant à:
(1) faire réagir un composé de formule **i** 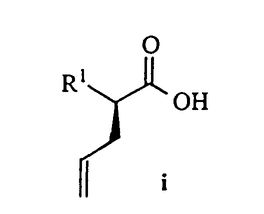 avec de l'iode pour obtenir de manière prédominante la *(3R, 5R)*dihydrofuranone de formule **iib** 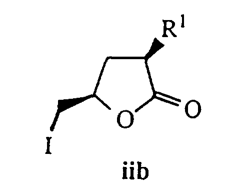
(2) faire réagir la 3*(R)*-3-R¹-5*(R)*-iodométhyldihydrofuran-2-one de l'étape (1) avec l'acide thioacétique et le méthoxyde de sodium pour obtenir un intermédiaire de formule **iiib** dans lequel W est un groupe acétyle, 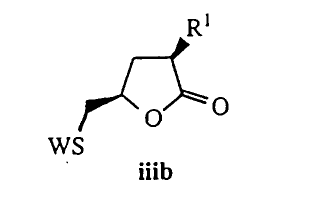
(3) déprotéger le soufre de la lactone **iiib** de l'étape (2), W étant un acétyle, par réaction avec le borohydrure de sodium dans le méthanol ou l'éthanol, pour fournir le composé **ivb',** 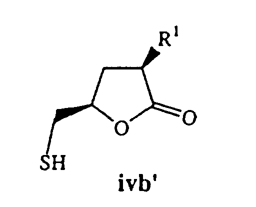 et
(4) protéger le thiol libre de l'étape (3) ci-dessus pour fournir un composé **ivb** dans lequel Z est le triphénylméthyle.

21. Procédé pour la préparation d'un intermédiaire de formule **iva** 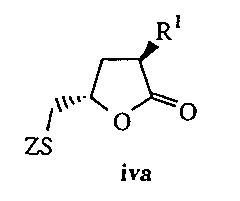 dans lequel R¹ est tel que défini dans la revendication 1 et Z est H ou un groupe de protection du thiol,
qui comprend les étapes consistant à:
(1) faire réagir un composé de formule **i** 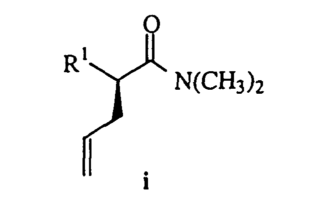 avec de l'iode pour obtenir de manière prédominante la (3*R*, 5*S*)dihydrofuranone de formule **iia** 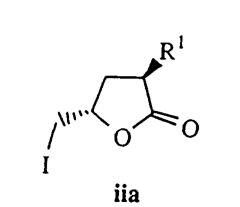
(2) faire réagir la 3*(R)*-3-R¹-5*(S)*-iodométhyldihydrofuran-2-one de l'étape (1) avec un réactif soufre, HSW et une base, W étant un groupe de protection du thiol, pour obtenir un intermédiaire de formule **iiia**, 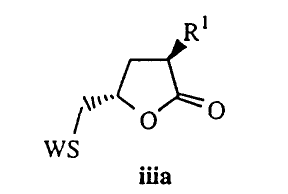
(3) déprotéger le soufre de la lactone **iiia** de l'étape (2) en conditions réductrices, par hydrolyse acide ou hydrolyse basique, pour fournir le composé **iva'**, 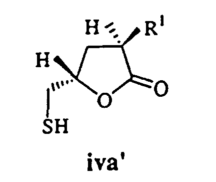 et
(4) protéger le thiol libre de l'étape (3) ci-dessus pour fournir le composé **iva** dans lequel Z est H ou un groupe de protection du thiol.

22. Procédé selon la revendication 21, pour la préparation d'un intermédiaire de formule **iva** 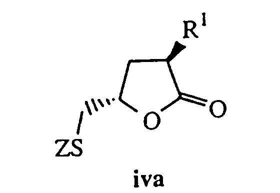 dans lequel R¹ est tel que défini dans la revendication 1 et Z est H ou un groupe de protection du thiol,
qui comprend les étapes consistant à:
(1) faire réagir un composé de formule **i** 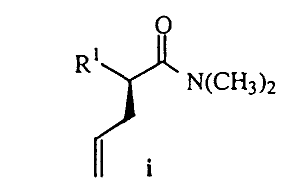 avec de l'iode pour obtenir de façon prédominante la (3*R*, 5*S*)dihydrofuranone de formule **iia** 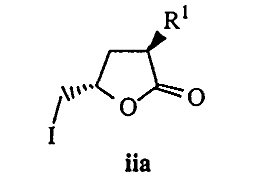
(2) faire réagir la 3*(R)*-3-R¹-5*(S)*-iodone de l'étape
(1) avec de l'acide thioacétique et du méthoxyde de sodium pour obtenir un intermédiaire de formule **iiia, W** étant un groupe acétyle, 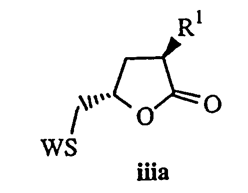
(3) déprotéger le soufre de la lactone **iiia** de l'étape (2), W étant l'acétyle, par réaction avec du borohydrure de sodium dans le méthanol ou l'éthanol, pour fournir le composé **iva'**, 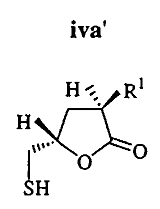 et
(4) protéger le thiol libre de l'étape (3) ci-dessus pour fournir le composé **iva** dans lequel Z est le triphénylméthyle.

23. Composé selon la revendication 1, qui est le ((1*S*)-2,2-diméthyl-1-méthylcarbamoylpropyl)amide de l'acide (2*R*)-2-((2*S*)-2-hydroxy-3-mercaptopropyl)nonanoïque ou le ((1*S*)-2,2-diméthyl-1-méthylcarbamoylpropyl)amide de l'acide (2R,4*S*)-4-hydroxy-2-isobutyl-5-mercaptopentanoïque.

24. Composition pharmaceutique comprenant un composé selon la revendication 1 ou la revendication 23 et un support pharmaceutiquement acceptable.
